# EUROPEAN PATENT APPLICATION

(11) **EP 3 901 617 A1**
(43) Date of publication of application: **27.10.2021**
(21) Application number: 20171328.6
(22) Date of filing: 24.04.2020
(51) Int. Cl.: G01N 21/65

(54) **IMPROVED BIOMOLECULE DETECTION USING SURFACE-ENHANCED RAMAN SPECTROSCOPY**

(71) Applicant: NOSTICS B.V., 2613 AX Delft (NL)
(72) Inventor: LABAN, Vincent Christiaan, NL-2613 AX Delft (NL); RENNEN, Eva Aleida Johanna, NL-2613 AX Delft (NL); NIEMEIJER, Rochella Alexia, NL-2613 AX Delft (NL)
(74) Representative: Arnold & Siedsma

(57) **Abstract**

The invention relates to the detection of communicable disease agents such as bacteria, archaea, protozoa, algae, fungi, viruses, prions, and multicellular parasites, and biomolecules therein, employing surface-enhanced Raman spectroscopy. Raman technology typically detects vibrational bonds between atoms in biomolecules and can provide a distinct Raman spectrum reflecting the distinct collection of those bonds that allows identification of said biomolecule. Each biomolecule has its own signature collection of bonds and each biomolecule or collection of biomolecules thus has its own signature Raman spectrum. The invention provides a chip conditioned for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof to detect this characteristic signature and identify signature collections of biomolecules of disease agents in clinical samples taken from a subject to determine health or disease status of a subject

## Description

### Field

The invention relates to the detection of a biomolecule or collections of biomolecules, in particular in biological samples, such as samples from cells, micro-organisms and communicable disease agents such as bacteria, archaea, protozoa, algae, fungi, viruses, prions, and multicellular parasites, and biomolecules therein, employing surface-enhanced Raman spectroscopy or surface-enhanced Raman scattering (herein identified as SERS) means and methods. Raman technology typically detects vibrational bonds between atoms in biomolecules and can provide a distinct Raman spectrum reflecting the distinct collection of those bonds, allowing specific identification of micro-organisms and communicable disease agents. Each biomolecule has its own signature collection of bonds and each biomolecules or collection of biomolecules thus has its own signature Raman spectrum. This characteristic signature may be used to identify and characterize collections of biomolecules of disease agents in clinical samples taken from a subject to determine health or disease status of a subject.

### Background.

Communicable disease agent surveillance is the ongoing, and preferably systematic, collection, sampling, testing, analysis, and interpretation of outcome-specific data for use in planning, implementing, and evaluating public, animal or plant health policies and practices. A communicable disease surveillance system serves two key functions; early warning of potential threats to public, animal or plant health status and communicable disease monitoring functions which may be disease-specific or multi-disease in nature. The early warning functions of surveillance are fundamental for public, animal and plant health security. Typically, detection of communicable disease agents starts with taken samples of a subject, often a subject suspected of carrying said agent, but also often sampling a random part of the population of subjects suspected of carrying said agent. Sample (often named clinical sample when collected from animals of man), such as a crude extract from tissues such as plant or animal tissue, or part of said tissues, or blood or urine samples, biopsy, throat swab, nose swab, vaginal swab, urethra swab, semen, sputum, saliva, smear, in short, is herein defined as any biological material collected from a subject in the course of conducting an investigation towards a cause of disease.

Recent outbreaks and even pandemics such as caused by the severe acute respiratory syndrome (SARS), Middle East respiratory syndrome (MERS), avian influenza, the new coronavirus (SARS-Cov-2) causing COVID-19, African Swine fever, hog cholera, foot-and-mouth disease, and potential threats from biological and chemical agents, demonstrate the importance of effective regional, national and global surveillance and response systems wherein disease testing is of high quality relating to test specificity and sensitivity. Fast and reliable tests for are urgently needed to bring outbreaks or pandemics under control as soon as possible. Most laboratories use a molecular method called reverse transcription polymerase chain reaction, or RT-PCR for short, to detect such nucleic acid of such agents. This is well established and can detect even tiny amounts of agent - but at the same time it can be time consuming and prone to error. In particular, the ongoing outbreak of the novel coronavirus disease (COVID-19) has spread globally and poses a threat to public health in more than 200 countries. Reliable laboratory diagnosis of the disease has been one of the foremost priorities for promoting public health interventions. The routinely used reverse transcription polymerase chain reaction (RT-PCR) is currently indeed the reference method for COVID-19 diagnosis. However, it also reported a number of false-positive or -negative cases, especially in the early stages of the novel virus outbreak. Recently, Qiu et al. ACS Nano 2020, doi.org/10.1021/acsnano.0c02439 developed an alternative test method in the form of an optical biosensor. In this work, a dual-functional plasmonic biosensor combining the plasmonic photothermal (PPT) effect and localized surface plasmon resonance (LSPR) sensing transduction is thought to provide an alternative solution for the clinical COVID-19 diagnosis.The sensor requires to combine two different effects to detect a virus: an optical and a thermal one. The sensor is based on tiny structures of gold, so-called gold nanoislands, on a glass substrate. The sensor requires artificially produced DNA receptors that match specific RNA sequences of the virus in question, SARS-CoV-2, that are grafted onto the nanoislands. This coronavirus is a so-called RNA virus: Its genome does not consist of a DNA double strand as in living organisms, but of a single RNA strand. The receptors on the sensor are therefore the complementary sequences to the virus' unique RNA sequences, which can identify the virus. The technology used for detection is called LSPR, short for localized surface plasmon resonance. This is an optical phenomenon that occurs in metallic nanostructures. An optical sensor than is used to measure changes in said phenomenon and determine whether the sample contains the RNA strands. However, it is important that only those RNA strands that match exactly the DNA receptor on the sensor are captured. This is where a second effect is needed for the sensor to specifically detect viral RNA. If the same nanostructure on the sensor is excited with a laser of a certain wavelength, it produces localized heat, and that heat serves to generate better matches between DNA-receptors and viral RNA.

Specificity in communicable disease testing and surveillance refers to the proportion of subjects without the disease that are correctly considered by the testing system as not having the disease. Specificity is expressed as (number of subjects without the disease as detected by the testing system / total number of subjects without the disease as tested by the testing system) x 100. It is desired to have a specificity of 100%, however, this is in practice hardly obtainable, think for example of taking a negative sample from an otherwise infected (positive) subject, said subject just by chance not presenting the agent at the place the sample was taken, or of human failure to sufficiently fill a sample tube.

Actual low-test specificity, however, invariably results in the surveillance system indicating many "false-positive" test results and thus "false" outbreaks and would for example necessitate public health care staff to spend a lot of resources to verify and investigate. For example, when specificity is determined to be as low as 90% (or even 95%), and 99.000 disease-free subjects are tested, 89.100 (94.050) subjects would be classified as not having the disease under study. However, 9900 (4950) perfectly-disease-free subjects would be wrongly classified as positive and would need to be retested, creating additional burden with increased workload and often havoc to testing routines in strenuous outbreak times.

Low specificity not only has detrimental logistic and financial health-care consequences, but moreover, particularly in human diseases such as SARS, flu or COVID-19, provides great (emotional and practical) burden to factual disease-free subjects that test disease-positive, need to be retested, and then test negative because of inaccurate testing in the first instance. Often such, in fact disease-free, subjects are unnecessarily but obligatory subjected to strict health and travel restrictions or even taken under precautionary quarantine because health-care staff and policies simply do not want to take risks of further transmission. Consequently, in particular in outbreak situations, when large human, animal or plant populations need be tested while the number of factual subjects with disease is considered small in comparison to the number of disease-free subjects, a high specificity of test and surveillance systems, preferably as close to 100% specificity as possible, is desired.

Sensitivity in communicable disease testing and surveillance refers to the proportion of actual cases in a population that are detected and notified through the system. Sensitivity is expressed as (number of subjects with the disease as detected by the testing system / total number of subjects with the disease as tested by the testing system) x 100. It is also desired to have a sensitivity of 100%, however, again, this is in practice hardly obtainable, think for example of inadvertently not placing a sample in the test system, just by chance of faulty identification or human error.

Sensitivity is particularly important in an early warning system designed to detect outbreaks and refers to the proportion of disease-cases detected and reported through the system. Low sensitivity would result in the surveillance system indicating many "false-negative" test results. For example, when surveillance sensitivity is determined to be as low as 90% (or even 95%), and 1000 diseased subjects are tested, 900 (950) subjects would be classified as having the disease under study. However, 100 (50) diseased subjects would be wrongly classified as negative and are initially missed. However, in practice these cases often turn up to be retested when disease symptoms of said subjects are found to persist, and then tests often show positive. Positive (diseased) cases tend to present themselves anyway, so to say. Therefore, sensitivity mainly refers to the proportion of cases detected and reported through the system.

The surveillance system often includes the use of "thresholds" which should trigger intervention. At threshold 1 (one subject is tested positive), retesting is good practice and most often always required, if only to confirm the disease in question. Disease status is then preferably also determined by an independent and more complete means of ascertainment, often using more testing systems than one. It is usually not practical to obtain highly accurate estimates of sensitivity of a system at large, as this requires the true number of cases in the population be known, something that is almost impossible. It is of upmost importance that the diagnosis of reported positive cases be confirmed to eliminate "false-positives" (caused by tests having <100% specificity).

In above used example of total of 100.000 subjects, of which 1000 are deemed positive, and settings for specificity and sensitivity levels are at 95% and 90%, respectively, close to 95% of subjects would be correctly classified. Such test specifications render a wrong classification to 5050 subjects, leaving health staff with far too many uncertainties to apply proper and swift control measures. Such specificity and sensitivity levels are simply not high enough for trustworthy outbreak management.

Therefore, in the stringent practice of communicable disease agent surveillance, it general follows that communicable disease agent testing specialists have a foremost interest in tests having a close-to-100% specificity, and can live with sensitivities of around 90-95% more easily than with tests that have close-to-100% sensitivity but show less specificity. This is especially the case for communicable disease agent testing in disease outbreaks in otherwise relatively disease-agent free populations where negative cases far outnumber positive cases, as is usually the case.

Analytical capabilities of Raman spectroscopy have changed fundamentally. Due to this technical development, relatively cheap, portable Raman devices for onsite analysis are available. Moreover, for some molecules in special metal nano-resonators, the efficiency of Raman scattering is so high, that, when using SERS-sensors (herein also called chips or substrates), a limit of detection even of the order of 10(-18) fold m(-3) can be achieved for some analytes.

Therewith, dramatically increased sensitivity and thus usage of SERS spectroscopy for analysis of industrial, biological, medical and environmental samples has recently been observed. However, while communicable disease agent testing through SERS has been employed under various experimental conditions, it has not yet found practical (let alone wide scale) application in communicable disease agent surveillance because of insufficient specificity, sensitivity or evidence that indeed many types of test (clinical) samples allow detection though SERS. Current Raman testing technology is not on par with the distinct specificity and sensitivity requirements as explained above.

For example, in US7889334B2, exemplifying Rotavirus detection with a SERS-biosensor, despite its enhanced detection capabilities with previous Raman technologies, only 81% of samples were correctly classified. Needless to say, such low percentage of classification does not suffice to help manage a Rotavirus outbreak during surveillance. Han et al., (Journal of Food Science Vol.75,Nr.5,2010), reports that extensive virus pre-purification is required for detection of food- or water-born norovirus, adenovirus, parvovirus, rotavirus, coronavirus, paramyxovirus, and herpesvirus, making said method useless for swift outbreak use on many samples. Kukushkin et al (Plos One, 2019; 14(4): e0216247) require specific aptamer-binding of influenza virus for SERS detection, making the test depend on the (undisclosed) selectivity and specificity of the aptamers selected. Similarly, Karn-orachai et al., (RSC Adv., 2016,6, 97791-97799) and Moore et al., (Biosensors (Basel), 2018 Jun; 8(2): 46.)aim to improve SERS-detection of virus using specific antibody probes to provide viral antigen binding, thus making the test depend on the selectivity and specificity of the antibody selected. Since SERS is useful for determining molecular structural information, and because SERS provides ultrasensitive detection limits including single molecule sensitivity, it has been used to detect bacteria and viruses using direct spectroscopic characterization or reporter molecule sandwich assemblies. However, this remarkable analytical sensitivity has, for the most part, not translated into the development of practical SERS systems for communicable disease agent testing, let alone in-situ diagnostic SERS systems for communicable disease agent testing. In essence, SERS-technology needs further improvement before it can be a useful instrument in communicable disease agent testing, let alone in communicable disease surveillance

### The invention

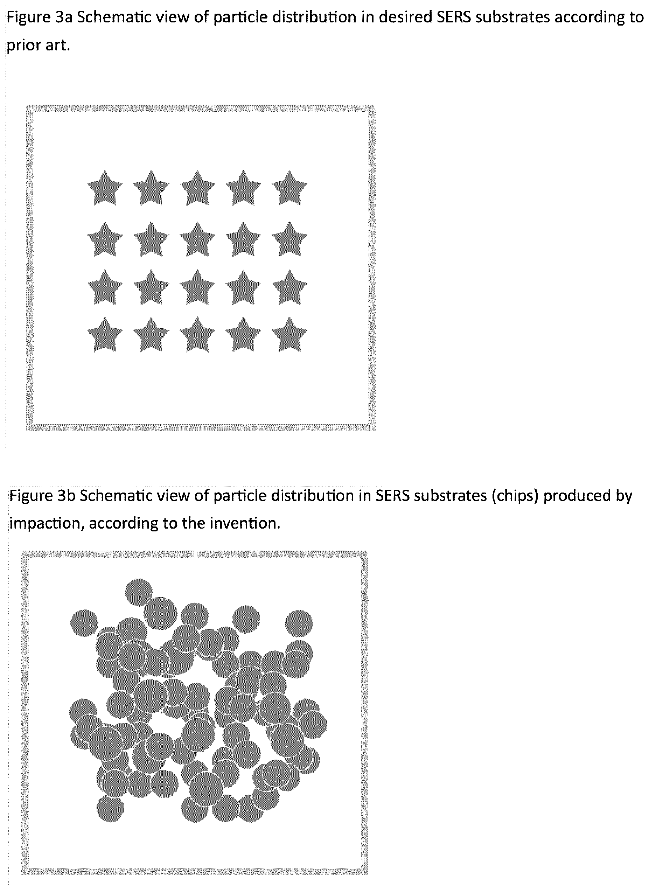

The invention provides a chip conditioned for surface-enhanced Raman spectroscopic detection of at least one biomolecule present in a sample, said chip having a support of which a solid surface at least partly is provided with a fractal-patterned multi-creviced sintered agglomerate of metal nanoparticles or composition of metal nanoparticles or metal compound nanoparticles on said surface. Examples of such a fractal-patterned multi-creviced sintered agglomerate are illustrated in figures 3, 4 and 5 herein. Said chip of the invention is at least partly conditioned with a surface comprising intricate, fractalized forms of nanoparticle agglomerates, with many sharp-edged nooks, crannies, fissures, pores and splits in many directions (herein identified as multi-creviced) which are thought to be generated through a diffusion-limited aggregation processes of said metal nanoparticles or compositions and compounds thereof, during deposition and sintering of said nanoparticles on said surface. It is in and around at those nooks, crannies, fissures, pores and splits of the nanoparticle surface where a multitude of so-called plasmon hot spots can reside that through their high frequent occurrence in said conditioned surface occurrence greatly enhance Raman detection sensitivities of even a single biomolecule. In essence, the closer the nanoparticles are situated together, the higher the SERS effect, due to increased hot spot formation allowing increased surface plasmon resonance. This is explained by the resonance gap or in general hot spots provided by the fractal-patterned multi-creviced sintered agglomerate of metal nanoparticles or composition of metal nanoparticles or metal compound nanoparticles on said surface, as detailed below. Therewith, particles with a smaller resonance gap provide better SERS effect than particles with a higher resonance gap. In the dense fractal-patterned multi-creviced sintered agglomerate of metal nanoparticles or composition of metal nanoparticles or metal compound nanoparticles on said surface as provided by the invention, most particles are touching and at least as close as at least 32 nm , preferably 16 nm, more preferably 8nm, more preferably 4 nm, more preferably to each other. In this case the particles have a very high hot spot density per square mm, allowing for various explanations detailing the obtained improved surface enhanced Raman signals as detected using a chip according to the invention.

First, due to the high frequency and multi-directionality of hot spot occurrences that these fractal-patterned multi-creviced sintered agglomerates of the invention facilitate, their plasmonic modes can be excited practically by any light beam, independently of its polarization and direction of propagation. Therefore, the mutual orientation of the target and light becomes much less important than in the case of low-frequent hot spot occurrence or in the case of directional hot-spot occurrence as seen with nanoparticles deposited through oblique angle deposition technologies resulting in geometrical structures that function relatively well only under strict light orientation and direction of propagation requirements. Second, it is natural to expect that when a molecule is deposited on this intricate fractal patterned multi-creviced agglomerate as provided by the invention, it may stick somewhere between or near the many of nooks, crannies, fissures, pores and splits provided. This means that the molecule will closely associate to an extremely favourable local environment (hot spots) characterized by strongly enhanced electric fields. And third, as it is well known, the Raman scattering enhancement factor scales roughly as |E(ω)|2|E(ω ± Ω)|2, where Ω is the vibrational frequency of the molecule. So, it is important to have a large electric field not only at excited frequency ω, but also at Raman shifted scattering frequencies ωR= ω ± Ω (Le Ru E.C.; Etchegoin P.G. Principles of Surface-Enhanced Raman Spectroscopy, Elsevier: Amsterdam, 2009). As SERS is a phenomenon associated with the enhancement of the electromagnetic field surrounding small objects optically excited near an intense and sharp plasmon resonance, it is of paramount importance that the enhanced fields not only excite the adsorbate (probe) but that the scattered radiation will again be enhanced. The two resonances with close frequencies described above can provide such a realization of enhancement of these two radiation events best when in near vicinity. Thus, it is very important that the hot spots induced by these resonances are not separated in space but practically coincide, as is facilitated with the fractal-patterned multi-creviced sintered agglomerate of the invention.

In a preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof which is obtainable, preferably obtained through spark ablation and/or impaction deposition. If required, several nanoparticle surface production methods may be used. Hot Wire synthesis is an in general a comparable way of producing ultra small, ultra pure nanoparticles from atomic cluster range up to 80 nm. This technique, however, has a general low (microgram/hour) production, and when scaled up to increase production output, nanoparticle size in general also increases, to the detriment of Raman detection requirements. Deposition by magnetron sputtering is an in general comparable way of producing ultra small, ultra pure nanoparticles from atomic cluster range up to 10 nm. However, it suffers from low (microgram/hour) production and is difficult to scale up. Continuous Arc ablation is an in general comparable way of producing ultra small, ultra pure nanoparticles but typically produces larger particles from typically 20nm and bigger, with high production output (g/hour). Further, albeit more remote, production possibilities are Electro/Plasma or Flame spray methods (typically producing particles from 20nm and bigger that have less Raman potential), with high production output (g/hour) but less control on purity. Sputtering, Physical Vapor Deposition and Chemical Vapor Deposition are methods to produce particles form 30nm and bigger, with high production output (g/hour) but even less Raman potential. In general, above listed technologies have either bigger particles then obtainable with spark ablation, so the dense nanostructured layers required are out of reach, or deposition capacity is not nearly enough, as compared to impact deposition, to produce viable substrates that can be used for molecule detection on large scale

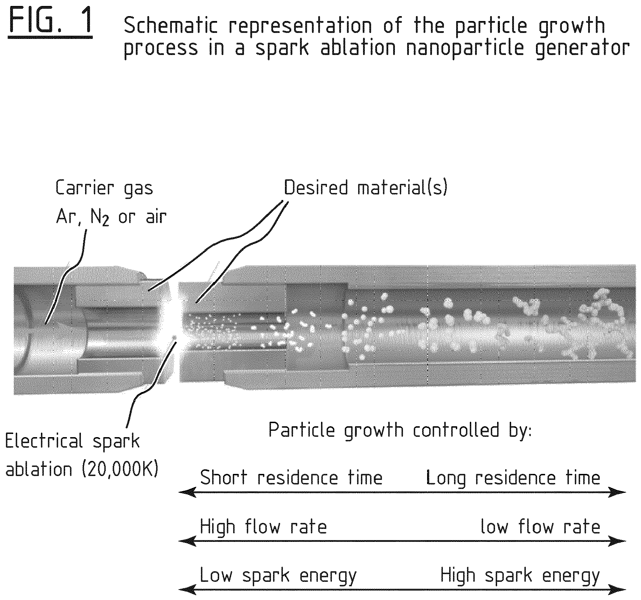

In a more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof which is obtained through spark ablation and deposition. The spark ablation process is depicted in figure 1 herein. Spark ablation (Tabrizi et al., Journal of Nanoparticle Research, volume 11, Article number: 315 (2009)) is a gas phase physical process for the generation of nanoparticles, by the continuous production of very small particles, and in certain cases deposition of those particles on various surfaces. Through spark ablation, the production of nanoparticles by microsecond spark discharge evaporation in inert gas is controllably achieved. The inputs of the nanoparticle production process are two conductive feedstock electrodes (the nanoparticle source material, often a rod with e.g. Ag, Au or Cu, or other nanoparticle source material, or mixtures thereof, preferably selected from a group of suitable elements as shown in Table 10, electricity, and a carrier gas, or mixtures thereof (preferably selected group of suitable elements as shown in Table 10). The output is a highly concentrated aerosol of pure (metal, metal compound or metal composite) nanoparticles suspended in a clean gas at low temperature (<50 °C). A typically useful nanoparticle agglomerate for use in the invention as provided herein may be produced using a nanoparticle generator such as VSP-G1 nanoparticle generator commercially available from the company VSParticle (VSPARTICLE B.V. Molengraaffsingel 10, 2629 JD Delft, The Netherlands, info @vsparticle.com), wherein the particle size may be controlled, as shown in figure 1. In a further preferred embodiment, the invention provides a chip according to the invention as obtainable, preferably obtained, by spark ablation wherein an average distance between nanoparticles or compositions thereof (or metal compound nanoparticles) is at most 32 nm, more preferably at most 16 nm, more preferably at most 8 nm, more preferably at most 4 nm, more preferably at most 2 nm, from each other is achieved. In yet another preferred embodiment, the invention provides a chip according to the invention as obtainable, preferably obtained, by spark ablation wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof which is obtainable, preferably obtained, through impaction deposition, as for example depicted in figure 3.2. By impaction, which generally occurs in a relative vacuum, a flow stream of aerosol nanoparticles occurs in an angle directed towards the solid substrate surface. Impaction speed may be accelerated by increasing vacuum. Said angle may be oblique in respect to the surface, in particularly if so desired, to result in oblique angle deposition via impaction. Straightforward deposition results are obtained when the stream is directed close to perpendicular, or fully perpendicular to the solid substrate surface. The nanoparticles impact and sinter onto the substrate and are randomly spread. As impaction is done in a vacuum, the vacuum draws the nanoparticles trough a critical orifice. The particles accelerate in the vacuum and impact on a target, in this case the chip as provided herein. Deposition time determines the thickness of the impacted layer but does only little effect the fractal-patterned multi-creviced sintered agglomerate of metal nanoparticles or composition (or metal compound nanoparticles) in the topmost layer. When the nanoparticles impact on the surface the particles emit heat, this is enough to sinter the particles and stick to other particles in its vicinity through atomic diffusion in between particles (Chitrakaret al., Acta Materialia (2017), doi: 10.1016/j.actamat.2017.05.062.)

In a further preferred embodiment, the invention provides a chip according to the invention as obtainable, preferably obtained, by impaction deposition wherein an average distance between nanoparticles or compositions thereof (or metal compound nanoparticles) is at most 32 nm, more preferably at most 16 nm, more preferably at most 8 nm, more preferably at most 4 nm, more preferably at most 2 nm, from each other is achieved. In yet another preferred embodiment, the invention provides a chip according to the invention as obtainable, preferably obtained, by impaction deposition wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In a most preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof which is obtainable, preferably obtained, through spark ablation followed by impaction deposition. As spark ablation as well as impaction events are closely and conveniently tunable in a nanoparticle generator equipped for spark ablation as well as for deposition by (preferably vacuum-assisted) impaction (such as is the case with a VSP-G1 nanoparticle generator as identified above, adapted with printer VSP-P1 which combines a VSP-G1 with a deposition vacuum chamber allowing to tune the specific requirements). Chip production according to the invention is preferably performed in such a dual-purpose system. Finetuning these events allows tuning particle distance as well as particle size.

In a further preferred embodiment, the invention provides a chip according to the invention as obtainable, preferably obtained, by spark ablation followed by impaction deposition wherein an average distance between nanoparticles or compositions thereof (or metal compound nanoparticles) is at most 32 nm, more preferably at most 16 nm, more preferably at most 8 nm, more preferably at most 4 nm, more preferably at most 2 nm, from each other is achieved. In yet another preferred embodiment, the invention provides a chip according to the invention as obtainable, preferably obtained, by spark ablation followed by impaction deposition wherein the size of said particles ranges from about 1 nm to at about 20 nm, more preferably ranges from about 1 nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In a more preferred embodiment, the invention provides a chip according to the invention as obtainable, preferably obtained, by spark ablation followed by impaction deposition wherein an average distance between nanoparticles or compositions thereof (or metal compound nanoparticles) is at most 32 nm is achieved and wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In a more preferred embodiment, the invention provides a chip according to the invention as obtainable, preferably obtained, by spark ablation followed by impaction deposition wherein an average distance between nanoparticles or compositions thereof (or metal compound nanoparticles) is at is at most 16 nm is achieved and wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In a more preferred embodiment, the invention provides a chip according to the invention as obtainable, preferably obtained, by spark ablation followed by impaction deposition wherein an average distance between nanoparticles or compositions thereof (or metal compound nanoparticles) is at most 8 nm is achieved and wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In a more preferred embodiment, the invention provides a chip according to the invention as obtainable, preferably obtained, by spark ablation followed by impaction deposition wherein an average distance between nanoparticles or compositions thereof (or metal compound nanoparticles) is at most 4 nm is achieved and wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm.

In a more preferred embodiment, the invention provides a chip according to the invention as obtainable, preferably obtained, by spark ablation followed by impaction deposition wherein an average distance between nanoparticles or compositions thereof (or metal compound nanoparticles) is at most 2 nm is achieved and wherein the size of said particles ranges from about 1 nm to at about 20 nm, more preferably ranges from about 1nm to at about 10 nm, more preferably ranges from about 3nm to at about 13 nm, more preferably ranges from about 3 nm to at about 10 nm more preferably ranges from about 1 nm to at about 7 nm, most preferably ranges from about 2 to about 5 nm. A particularly useful particle size is at around 4nm. Another particularly useful particle size is at around 2nm. The closer the nanoparticles are together, the higher the SERS effect.

This is explained by the resonance gap or in general hot spots. Particles with a smaller resonance gap have better SERS effect than particles with a higher resonance gap. In the dense nanostructured layer, we may have particles that are as close as 1nm to each other. In that case the particles have a very high hot spot density per square mm are result in very high sensitivity SERS.

In a another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained by spark ablation followed by impaction deposition, wherein said metal is selected from the group of silver, nickel, aluminium, silicon, gold, platinum, palladium, titanium, copper, cobalt, zinc, or selected from an alloy of at least two metals selected from said group, or wherein said composition (or metal compound) comprises an oxide, nitride, silicide, phosphide, oxynitride, or carbide of said metal. In a more preferred embodiment, said metal is selected from the group of silver, gold, platinum, copper, or selected from an alloy of at least two metals selected from said group, As alloy, a particularly preferred alloy is selected from silver mixed with up to 5%, preferably <2% platinum allowing production of fractal-patterned multi-creviced sintered agglomerate of metal nanoparticles, another particularly preferred alloy is selected from silver mixed with up to 10% copper, preferably <5% copper, allowing production of fractal-patterned multi-creviced sintered agglomerate of metal nanoparticles. In a further preferred embodiment, close to 100% silver is preferred allowing production of fractal-patterned multi-creviced sintered agglomerate of metal nanoparticles.

In a another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, wherein the support is selected from the group of silica, alumina, polymer (such as thin transparent polymer film or plastic) and paper, and combinations thereof. In a most preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, wherein the support is glass, such as quartz, zirconium or gorilla (alkali-aluminosilicate) glass.

In a another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, wherein an Indium Tin Oxide (ITO) top layer is deposited upon which said agglomerate. Thickness of said ITO layer preferably is at about 10- 200 nm, more preferably 50 to 150 nm, most preferably at around 100nm. Said ITO layer may serve as an additional enhancer of Raman signal, due to the improved strength of the surface plasmon resonance spectra, and the larger surface electric field strength compared to other surfaces like quartz

In a another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, wherein said agglomerate is deposited in a spot surface size of at least 1 square millimeter, preferably at least 4 square millimeters, more preferably at least 9 square millimeters. Said spot preferably is provided with a fractal-patterned multi-creviced sintered agglomerate of metal nanoparticles or composition (or metal compound nanoparticles) according to the invention with a layer of said agglomerate with thickness preferably between 100 - 1500 nm, more preferably between 200 and 1100 nm, more preferably between 300 and 800 nm, most preferably between 450 and 650 nm. Such spot surfaces sizes and thickness are in particular preferred when Raman devices with static beam lasers are used. In particular Raman devices employing Orbital Raster Scan (ORS or 'raster'), collect spectra from several points on the substrate in a circular movement, following a raster design. ORS use in particular prevents (over)heating the sample and thereby influencing the quality of the sample and the measurement. With ORS, spot surface size of at around 3x3 mm is preferred. Whenever laser requirements would change and larger spots would be required, spot surface size of a chip according to the invention can be enlarged without any difficulty by setting the deposition parameters during the chip production process accordingly.

In a another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, wherein the support is provided with a sufficient large surface dimension, preferably at least 10x10 millimetre, or larger. Other suitable chip support formats vary from 5x5 mm via 15x20 mm or 25x25 mm to 20x30 mm (as for example obtained through Ossila; https://www.ossila.com/pages/support) or are equipped with elongated (pan handle like) stems for improved handling experience. The chip can either be larger or smaller to be compatible with chosen chip holder attached to chosen spectrometer and/or be an integral part of a chip holder that is attached to said chip and can be connected to the spectrometer. In a another more preferred embodiment, the invention provides a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, wherein said at least one biomolecule is derived from a communicable disease agent, such as a virus or bacterium.

Biomolecules can be defined as any molecules, such as peptides and proteins, nucleic acids, lipids or carbohydrates, polysaccharides, cell wall components, that may be generated or produced by a living organism, and typically are unique for all distinct types or species of living organisms, and typically are unique for biomolecules generated or produced by organisms harboring an agent of communicable disease, such organisms carrying bacteria, archaea, protozoa, algae, fungi, viruses, prions, or multicellular parasites. Raman technology typically detects vibrational bonds between atoms in biomolecules and can provide a distinct Raman spectrum reflecting the distinct collection of those bonds. From a chemical perspective the molecular precursors generally are carbon, hydrogen, oxygen, nitrogen, phosphor and sulfur. These atoms specifically associate through bonding to small biomolecules that are the building blocks of life: distinct and widely varied nucleotides, amino acids, fatty acids and monosaccharides. As each biomolecule has its own signature collection of bonds, each of said distinct building blocks has its own signature Raman spectrum. This characteristic signature, and characteristic collection of signatures, is used herein to identify and characterize collections of biomolecules of distinct disease agents in clinical samples taken from a subject, therewith provides a distinct Raman spectrum of said agent, the SERS detected absence or presence, respectively, therewith sufficient to determine health or disease, respectively, status of a subject.

In general, the macromolecules itself tend to form larger biopolymers. These biopolymers belong to four major groups of essential components of prokaryotic as well as eukaryotic life: nucleic acids, proteins, lipids and carbohydrates. Besides, there are additional molecules with biological relevance that are synthesized within cells, like small or complex metabolites. The detection of biomolecules is essential in many analytical, medical, biochemical and pharmaceutical application fields. The term "biomolecule" is herein defined as a chemical substance generated by a living organism and includes amongst others nucleic acids, peptides/proteins, and their building blocks, which essentially may comprise one single biomolecules as well as a collection of biomolecules characteristic of a living organism or of an agent of communicable disease. A variety of reliable SERS detection schemes assigning Raman peak occurrences to the many types of vibrational bonds in the building blocks of life have already established.

Detection of biomolecules via Raman spectroscopy is always based upon detection of said distinct biomolecule associated peaks in Raman spectra obtained from such biomolecules. To illustrate, individual peak assignments of all 20 proteinogenic L-amino acids are known (e.g. Negri and Schultz, Online SERS detection of the 20 proteinogenic L-amino acids separated by capillary zone electrophoresis. Analyst. 2014 Nov 21;139(22):5989-98. doi: 10.1039/c4an01177e, incorporated herein by reference), and others.

Similarly, peak assignments of bonds of nucleic acids have been provided. Nucleotides are composed of a sugar, a phosphoryl group and a nucleobase, which can be a purine or a pyrimidine. There are two purine bases, adenine (A) and guanine (G) and three pyrimidine bases, cytosine (C), thymine (T) and uracil (U). Ribonucleic acid (RNA) and Deoxyribonucleic acid (DNA) are the polymers of nucleotides, which are designated by the type of the sugar in the backbone. In the case of RNA the sugar is D-ribose and for DNA it is 2-deoxy-D-ribose. To illustrate further, individual peak assignments have been provided for nucleobases purine and pyrimidine (Carrillo-Carrion et al., Determination of Pyrimidine and Purine Bases by Reversed-Phase Capillary Liquid Chromatography with At-Line Surface-Enhanced Raman Spectroscopic Detection Employing a Novel SERS Substrate Based on ZnS/CdSe Silver-Quantum Dots, incorporated herein by reference), and others. Also, individual peak assignments of adenine, guanine, cytosine, thymine, and uracil are available as well (Madzharova et al., Surface-Enhanced Hyper-Raman Spectra of Adenine, Guanine, Cytosine, Thymine, and Uracil, J Phys Chem C Nanomater Interfaces. 2016 Jul 21;120(28):15415-15423. doi: 10.1021/acs.jpcc.6b02753, incorporated herein by reference), and others.

Furthermore, Czamara et al., (Raman spectroscopy of lipids: A review Journal of Raman Spectroscopy 46(1) · December 2014, incorporated herein by reference), and others, provide peak assignments of bonds in lipids, and Wiercigroch et al (Raman and infrared spectroscopy of carbohydrates: A review, May 2017 Spectrochimica Acta Part A Molecular and Biomolecular Spectroscopy 185 DOI: 10.1016/j.saa.2017.05.045, incorporated herein by reference), and others, have characterized bonds in carbohydrate biomolecules via SERS. Thus, of the four major biomolecule buildings blocks, a wide variety of distinct Raman assigned peaks are available that sufficiently allow characterize with SERS of a wide variety of bonds in at least one distinct biomolecule, or in a distinct collection of biomolecules via SERS, or in distinct disease agents, or parts thereof, composed of such a collection of biomolecules, as such.

The invention also provides SERS detection of communicable disease agents such as bacteria, archaea, protozoa, algae, fungi, viruses, prions, and multicellular parasites, and biomolecules therein, employing surface-enhanced Raman spectroscopy or surface-enhanced Raman scattering means and methods, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition .

Also, the unique chemical bond composition and therewith unique SERS signature peak assignment of bonds therein, of each bacterium enables us to differentiate between bacteria, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each archaeon enables us to differentiate between archaea, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each protozoon enables us to differentiate between protozoa, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each alga enables us to differentiate between algae, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each fungus enables us to differentiate between fungi, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each virus enables us to differentiate between viruses, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each species-specific prion enables us to differentiate between prions, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

Also, the unique chemical bond composition and therewith unique SERS signature peak assignment of bonds therein, of each multicellular parasite enables us to differentiate between multicellular parasites, or any other agent, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition.

As herein described, the invention provides means and methods to specifically recognize a communicable disease agent such as SARS-Cov-2 virus (COVID-19), Influenza virus (H1N1, H3N2), Zika, nCov-NL63 virus and more, which is based on the chemical bond composition of these disease agents . The unique chemical bond composition of each virus enables differentiation between viruses, hence increasing the specificity of this diagnostic technology, employing a chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition. What is more, as the chemical bond composition of each communicable disease agent is a fingerprint that can be identified on the basis of the characteristics of the chemical component, for many agents said chemical composition is at least partly known and can at beforehand be compared between agents, such as in the case of the chemical structure of SARS-CoV-2, SARS-CoV and MERS-CoV and other corona viruses of which nucleic acid and protein sequence comparisons are described by Tahir ul Qamar, (Journal of Pharmaceutical Analysis, 26 March 2020, https://doi.org/10.1016/j.jpha.2020.03.009, incorporated herein by reference), as an example allowing further fine tuning of SERS technology as provided herein towards highly specific diagnostics.

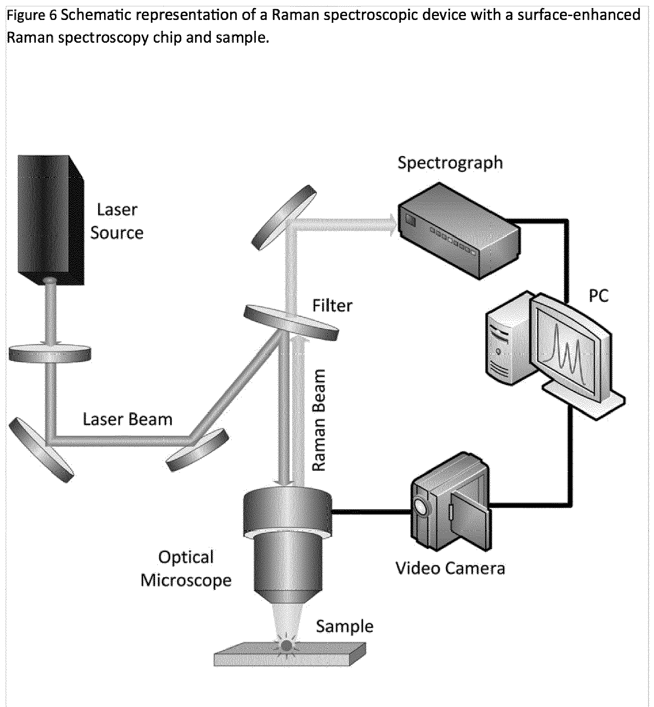

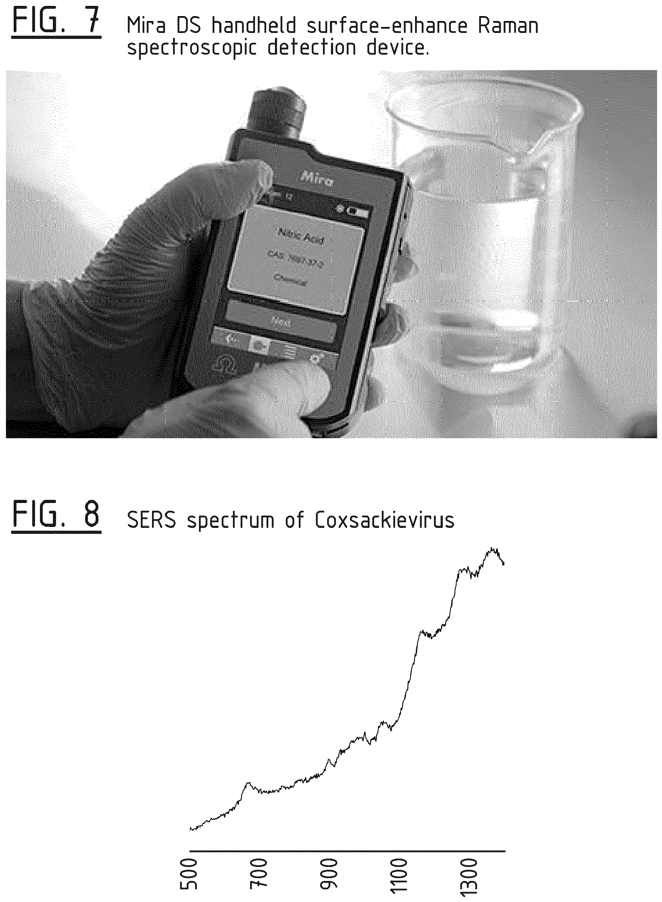

In another embodiment, the invention provides a detection system for detecting at least one biomolecule present in a sample, comprising a detection chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition, said chip suitable for carrying the sample, and means for measuring a Raman scattered light signal or spectrum of the sample on said detection chip, with means for collecting and processing the measured light signal or spectrum, and comparing the analysed data with data characteristic for the biomolecule. Such a detection system may employ Raman spectrometers that use lasers having a variety of wavelengths, enhancing the versatility of the invention as described herein. Most widely used excitation wavelengths for raman spectroscopy of biological samples are 532, 632, 647, 785 and 1064 nm (Synytsya et al, J. Raman Spectrosc. 2014, 45, 903-911). Usually a distinct choice of laser wavelength is made to avoid fluorescence, as sometimes fluorescence can give a stronger signal than the Raman scattering, resulting in a more or less weak detectable spectrum. Choosing or selecting a different, often longer, wavelength can often solve this problem. Other reasons for choosing specific wavelengths is because it may optimize sample behaviour. To perform measurements, a Raman spectrometer is required involving a receiver besides the laser described (see also figure 6 for schematic system requirements). Here as well the invention as described herein is versatile, a receiver can principle be any type of spectrometer useful for Raman detection, such as a desktop Raman device inVia confocal Raman Spectroscope from Renishaw, a portable Raman device such as Ava-Raman-B or -D from Avantes, or a handheld device such as the Metrohm Mira DS / P as shown in figure 7 may be applicable. For the purpose of rapid detection and localized use of the method according the invention, a handheld device may be preferred. When homeland security applications of the invention are desired at a certain location, it may be useful to use the same type of device as already is available for other homeland security SERS applications performed or desired to be performed at said location. Said means for determining based on the compared data the presence of absence of the biomolecule in the sample is preferably achieved in a computerized fashion, enabling rapid comparison with computerized and database-stored Raman spectra of communicable disease agents. Preferably, the invention provides said Raman spectroscopic device for measuring a Raman scattered light signal or spectrum as provided with a chip according to the invention, wherein said device is also provided with a database provided with Raman shift peak assignments, said assignments preferably selected from table 9, more preferably selected from an assignment or assignments provided for a virus, most preferably selected for a coronavirus.

The invention also provides a kit of parts (as for example schematically identified in figure 6 herein, said useful for detecting at least one biomolecule present in a sample, comprising a detection chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition for carrying the sample, a Raman spectroscopic device for measuring a Raman scattered light signal or spectrum of the sample on said detection chip, preferably handheld or portable. a computerized device for collecting and processing the light signal or spectrum, and comparing the analyzed data with data from a database carrying Raman peak assignments characteristic for one or more biomolecules of an agent under study, and an assessment device for determining based on the compared data the presence of absence of the biomolecule in the sample, preferably after which confidence indication is given upon the match of with a biomolecule. The invention also provides a kit of parts for detecting at least one biomolecule present in a sample, a detection chip for surface-enhanced Raman spectroscopic detection of at least one biomolecule present in a sample, said chip having a support of which a solid surface at least partly is provided with a fractal-patterned multi-creviced sintered agglomerate of metal nanoparticles or composition (or metal compound nanoparticles) thereof, as preferably obtainable or obtained, by spark ablation followed by impaction deposition for carrying the sample, a Raman spectroscopic device for measuring a Raman scattered light signal or spectrum of the sample on said detection chip, and a device for collecting and processing the light signal or spectrum and comparing the analyzed data with data characteristic for the biomolecule and determining based on the compared data the presence of absence of the biomolecule in the sample. To prepare sample to be deposited on said chip according to the invention, retrieved sample with possible virus may be inactivated by heat and/or chemicals dependent on agent (eg virus-) specific requirements, or remains active if safety protocols allow it, then diluted preferably in ethanol, methanol or demi water in a 1:100 ratio. A method for testing a sample for the presence of a biomolecule in a detection system according to the invention is thus provided comprising obtaining a biological sample, preferably a human sample, more preferably a nose or throat swab sample, dissolving this sample in a medium placing an aliquot of the diluted sample on said chip on top of said fractal-patterned multi-creviced sintered agglomerate of metal nanoparticles and/or metal compound nanoparticles, subsequently placing the chip in said means for measuring a Raman scattered light signal or spectrum of said system, subjecting said aliquot to light, determining the light signal or spectrum of said aliquot and comparing the analysed data with a data characteristic for the biomolecule, allowing determination in said sample the presence of the biomolecule, wherein the medium is "volatile" medium, such as 70% ethanol, and drying the diluted sample after placing the aliquot on the chip, and preferably the said testing is achieved in less than 60 minutes, facilitating homeland security use. An alternative method according to the invention is also provided wherein the medium is an "aqueous nonvolatile", such as demi water.

Sample is applied on chip covering the SERS active area. In the case of ethanol, drying time is preferred to evaporate the ethanol (unless sample is also compared with ethanol spectra in the device), in the case of water, wet measurements are preferred. Chip is placed in holder or chip with integrated holder is attached to spectrometer. Acquisition time is preferably 10 seconds per measurement, laser power set to 20mW. Data from the spectra is analyzed and a confidence indication is given. Total time from dilution to detection outcome is less than 10 minutes. As detailed in Table 11, handling and detection times per sample can be as rapid as 75 seconds per sample, and said handling and detection speed allows for rapid testing of larger numbers of samples, such as required in screening procedures. Spinning the samples before application on the surface is also possible.

The invention also provides a method for testing a sample in a detection system according to the invention, comprising obtaining a biological sample, preferably a human sample, more preferably a nose or throat swab sample, diluting this sample in a volatile medium, such as 70% ethanol, taking an aliquot, preferably of about 0.001 ml to 0.025 ml, more preferably of about 0.05 to 0.015 ml from said diluted sample, placing said aliquot on said chip on top of said fractal-patterned multi-creviced sintered agglomerate of metal nanoparticles and/or metal compound nanoparticles, drying the sample for at least 5, more preferably at least 10 minutes, subsequently placing the chip in said means for measuring a Raman scattered light signal or spectrum of said system, subjecting said aliquot to light, determining the Raman spectrum of said aliquot and comparing said aliquot spectrum with a database of Raman spectra, allowing determination in said sample the presence of a biomolecule with the Raman spectrum signature specific for a biomolecule. Such method as provided herein, wherein said testing may achieved in less than 60 minutes, preferably less than 30 minutes, may greatly facilitate homeland security use, by for example testing at borders or airports where authorities have Raman devices available.

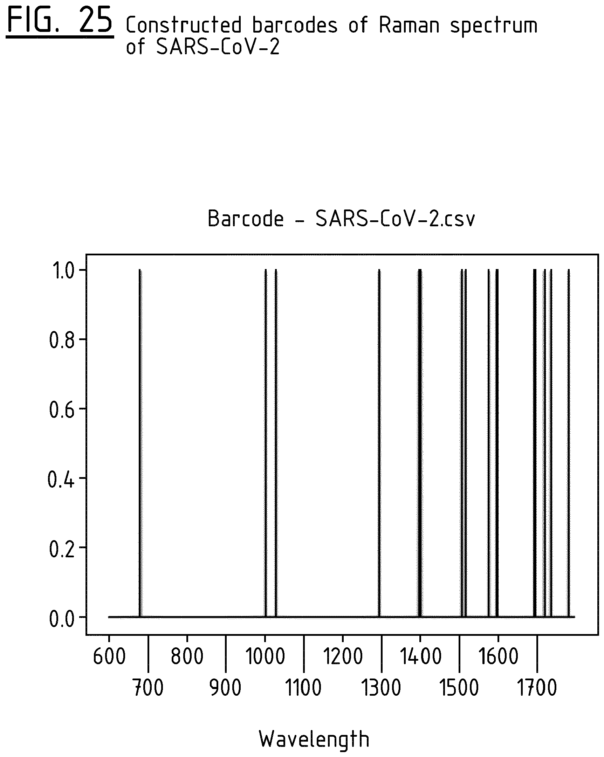

The invention also provides a method for testing a sample in a detection system according to the invention, comprising obtaining a biological sample, preferably a human sample, more preferably a nose or throat swab sample, diluting this sample in a medium, such as demi water, taking an aliquot, preferably of about 0.001 ml to 0.025 ml, more preferably of about 0.05 to 0.015 ml from said diluted sample, placing said aliquot on said chip on top of said fractal-patterned multi-creviced sintered agglomerate of metal nanoparticles and/or metal compound nanoparticles, subsequently placing the chip in said means for measuring a Raman scattered light signal or spectrum of said system, subjecting said aliquot to light, determining the Raman spectrum of said aliquot and comparing said aliquot spectrum with a database of Raman spectra, allowing determination in said sample the presence of a biomolecule with the Raman spectrum signature specific for a biomolecule. Such a method as provided wherein said testing may be achieved in less than 45 minutes, preferably less than 25 minutes, may greatly facilitate medical screening use, by fore example testing at intensive care units. The invention also provides a Raman spectroscopic device, preferably a handheld device, for measuring a Raman scattered light signal or spectrum provided with a chip according to the invention, preferably said device is also provide with a database provided with constructed barcodes of Raman spectrum as shown in any of figures 25, 26 and 27.

The invention also provides a method for making a chip conditioned for surface-enhanced Raman spectroscopic detection of at least one biomolecule present in a sample, said chip having a support of which a solid surface at least partly is provided with a fractal-patterned multi-creviced sintered agglomerate of metal nanoparticles and/or metal compound nanoparticles, said method comprising generating a flow of about 1 to 30 l/min of said nanoparticles in carrier gas in a vacuum of preferably 0.5, more preferably 0.2 mbar, at room temperature, preferably from 18 to 23 degrees Celsius, with a nanoparticle generator, preferably a VSP-G1 generator, provided with two metal electrodes with spark power from at about 0.1kV to about 1.3 kV and from at about 1 mA to at about 10 mA and depositing said nanoparticles on a solid surface with a nanoparticle printer attached to said generator, preferably a VSP-P1 nanostructured materials printer, wherein said deposition or printing occurs by impacting said nanoparticles on said surface trough directing said flow in an angle perpendicular to said surface, more preferably said gas being Argon, said flow for deposition set at a speed of 1 l/min, said spark power set at 1.3 kV and 10 mA; and said metal silver, preferably said surface being an on ITO (indium tin oxide) coated glass slide, preferably with dimension 20x15 mm The invention also provides a chip obtainable or obtained with a method according to the invention and use of a chip according to the invention in a method according to the invention.

### Figure legends

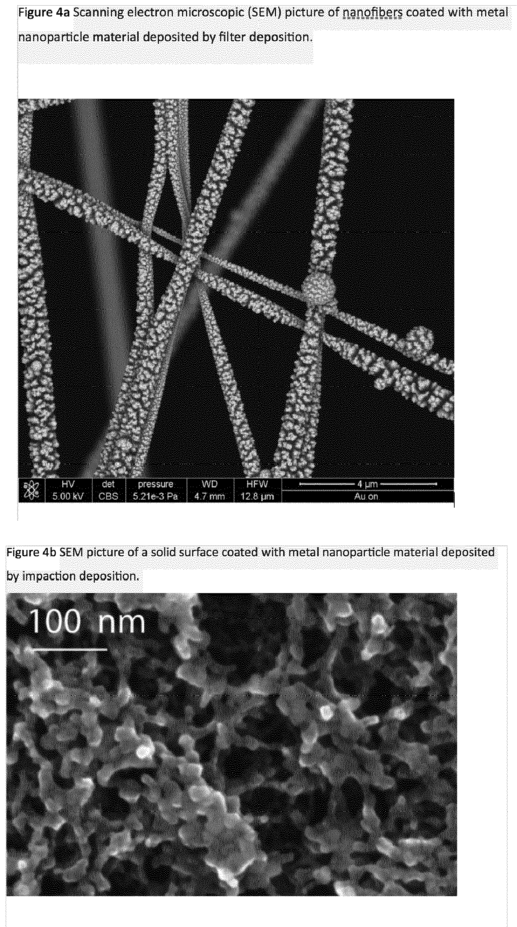

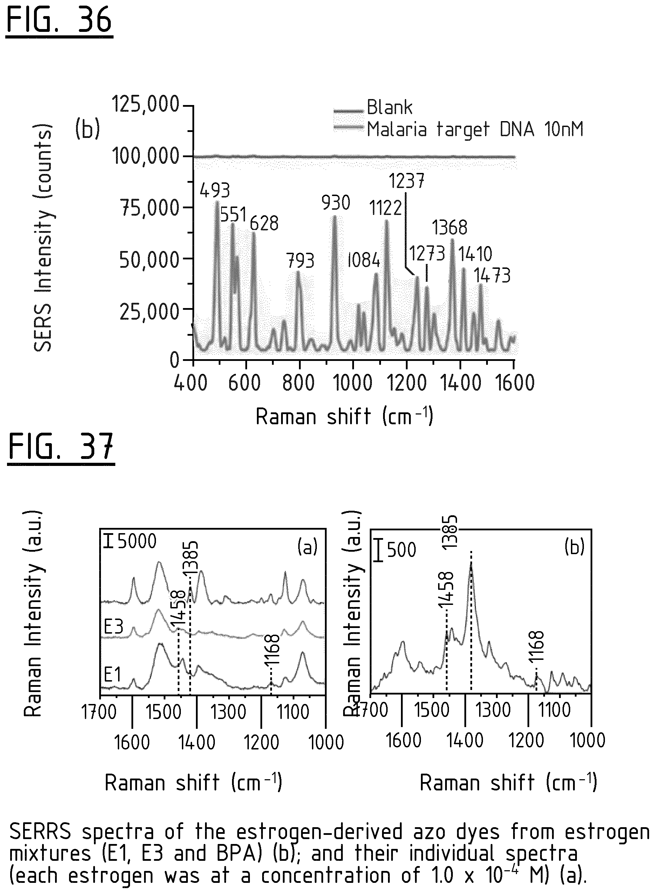

Figure 1. Schematic representation of the particle growth process in a spark ablation nanoparticle generator. A carrier gas flows through the device (left to right) passing the electrodes of the material desired to be deposited. Between the electrodes there is an electrical spark that ablates material (evaporating it and reducing it to particles with sizes such as several atoms) from the surface(s) of the electrodes in a controlled batch process. Particle growth is controlled by the residence time of the particles in the tube before deposition. Initial parameters influence the particle concentration and residence time: flow rate of the gas (high rate reduces residence time, low rate increases residence time) and the spark intensity (Voltage and current).
Figure 2. Schematic overview of various nanoparticle deposition methods after nanoparticle production with a nanoparticle generator: diffusion, impaction, and filtration (left), with transmission electron microscopic (TEM) image of deposited nanoparticles.
Figure 2.1. Top panel: Diffusion
   Flow stream of aerosol nanoparticles occurs parallel to the solid substrate surface. The nanoparticles diffuse onto the substrate and are randomly spread. This method of deposition or printing nanoparticles may create a smooth surface coverage, a monolayer. These smooth surfaces were not found to improve SERS applications.

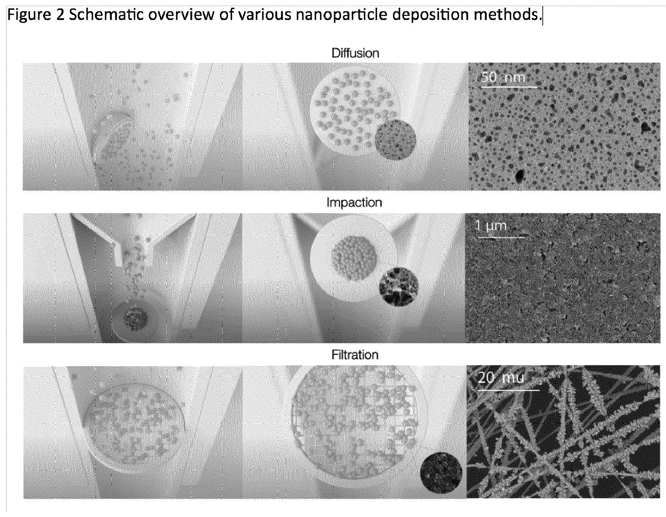

Figure 2.2. Middle panel: Impaction
   Instead of parallel, flow stream of aerosol nanoparticles occurs in an angle directed towards the solid substrate surface. Said angle may be oblique in respect to the surface, in particularly if so desired, to result in oblique angle deposition via impaction. Straightforward deposition results are obtained when the stream is directed close to perpendicular, or fully perpendicular to the solid substrate surface. The nanoparticles impact and sinter onto the substrate and are randomly spread. This method of deposition or printing nanoparticles is thought to create the desired rough surface coverage with desired hot spot morphology in a layer of nanoparticles. Through impaction, nanoparticles tend to aggregate into randomly sintered nanoparticles in a layer of fractal-patterned multi-creviced sintered agglomerates of metal nanoparticles or composition (or metal compound nanoparticles), as can be observed in the TEM picture at right, and also in figure 4. Such fractal-edged, multi-creviced surfaces as obtained by impaction were ultimately found herein to highly support and improve SERS applications in biomolecule detecting, in particular of a communicable disease agent.
Figure 2.3. Bottom panel: Filtration or filter deposition.
   Flow stream of aerosol nanoparticles occurs roughly perpendicular to a porous substrate surface, said surface composed like a sieve or filter. The nanoparticles alternatively deposit onto the solid filter matrix of the substrate surface or pass through its pores. This method of deposition or printing nanoparticles may create a surface structure similar to a sieve mesh with a network of nanoparticles loaded on branching matrix structures around pores or apertures. These sieve-like nanoporous surfaces do not support or improve SERS applications in detecting a communicable disease agent. Sensitive SERS detection is in practice not provided through filter deposition due to the inhomogeneity of the surface layer to reach a sufficiently high number of hotspots. With filter deposition, deposits are only built up on the walls of fibers or filter-matrix structures, a process that results in nanoporous surfaces. This reduces the required density and homogeneity of the layer in comparison to impaction deposition and does not provide a sufficiently dense and homogenous SERS signal to allow for specific or reproducible detection of Raman spectra.
Figure 3a Schematic view of particle distribution in desired SERS substrates according to prior art. In this view of the desired morphology of SERS substrates, desired particle distribution is illustrated with nanoparticles of all exactly the same size structured in an ordered way. Preferred is the size of around 50 nm or greater, and a small distance between the particles, around 10 nm or smaller. From literature it may until now be considered this is the ideal SERS substrate for the maximum enhancement.
Figure 3b Schematic view of particle distribution in SERS substrates (chips) produced by spark ablation and impaction, according to the invention. Particle size range is preferably 0-20 nm. Particle distance is preferably around 5 nm or smaller.
Figure 4a Scanning electron microscopic (SEM) picture of nanofibers coated with metal material deposited by filter deposition to 1) further illustrate the inhomogeneity of the surface obtained by filter deposition limiting SERS applications in the case of filter deposition, and 2) further illustrate the type of fractal clustering obtained through sintering.
Figure 4b Figure 4b SEM picture of a solid surface coated with metal nanoparticle material deposited by impaction deposition.

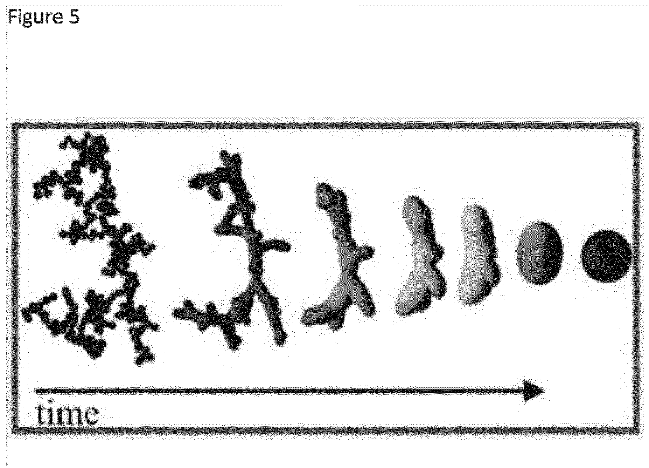

Figure 5 Two-dimensional picturing of the sintering process. Once sintering starts between constituent primary particles, particle coalescence proceeds via fractal cluster formation when sinter necks (points of atomic diffusion and coalescence are formed between particles converting collections of individual particles into fractal-edged, multi-creviced agglomerates (indicated at far left) that, preferably when constituent particles are sized between 1 to 20 nm, and impact at around room temperature, typically support strongly increased detection in surface-enhanced Raman spectroscopy by allowing increased plasmon hot spot formation.
   Further increased sintering in time, as is for example occurring when the particles impact on surfaces with increased temperatures closer to particle melting point, then increasingly coalesces the agglomerates to aggregated coalescent metal particles of increasingly larger sizes, in the process losing much or all of their fractal-patterned multi-creviced character and therewith losing SERS functionality. During even further prolonged sintering, the aggregates progressively densify until complete compact structures are formed, after long process times at high temperatures, losing SERS functionality altogether.
Figure 6 Schematic representation of a Raman spectroscopic device with a surface-enhanced Raman spectroscopy chip and sample. Required for the technical setup is a laser source (left) with a line filter, mirrors, focusing optics (can optionally be used in combination with an optical microscope and video camera but not necessary) and the chip with the sample depicted bottom center. The emitted Raman scattering from the sample passes a filter, that blocks the laser, towards the detector (spectrograph). The detector receives the Raman signal and passes it on to a computer with database (computerized device with database) where the spectrum is displayed, processed, and/or analyzed, in comparison with database-stored spectra
Figure 7 Mira DS handheld surface-enhance Raman spectroscopic detection device.
Figure 8: SERS spectrum for inclusion in a database as provided herein of Coxsackievirus is shown. The spectrum of Coxsackievirus has distinct characteristic Raman peak shift assignments as shown in table 1.

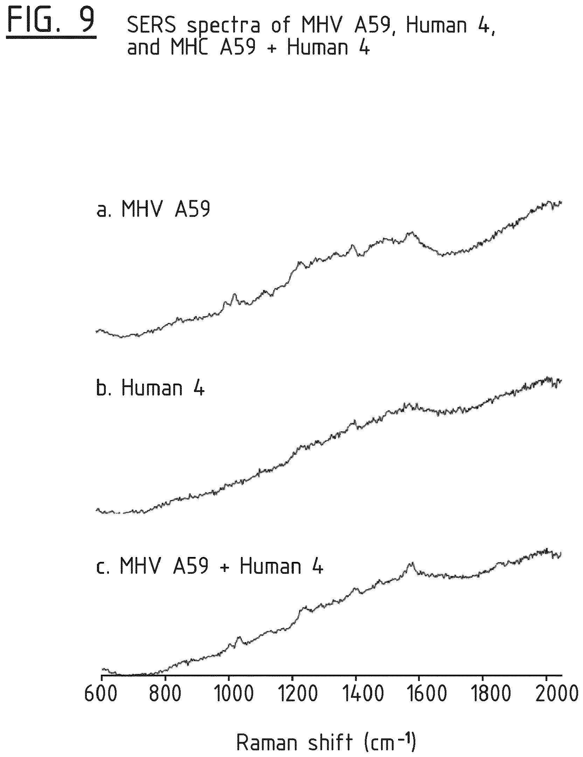

Figure 9. SERS spectra for inclusion in a database as provided herein of MHV A59, human 4, and MHC A59 + Human 4 is shown. MHV A59 has characteristic Raman peak shift assignments as shown in table 2. Raman spectra of the MHV A59 spiked human sample have similar peak assignment as the MHV A59 Raman spectra, as tested alone, therefore it can be said that a human negative sample does not hold back viral signals.

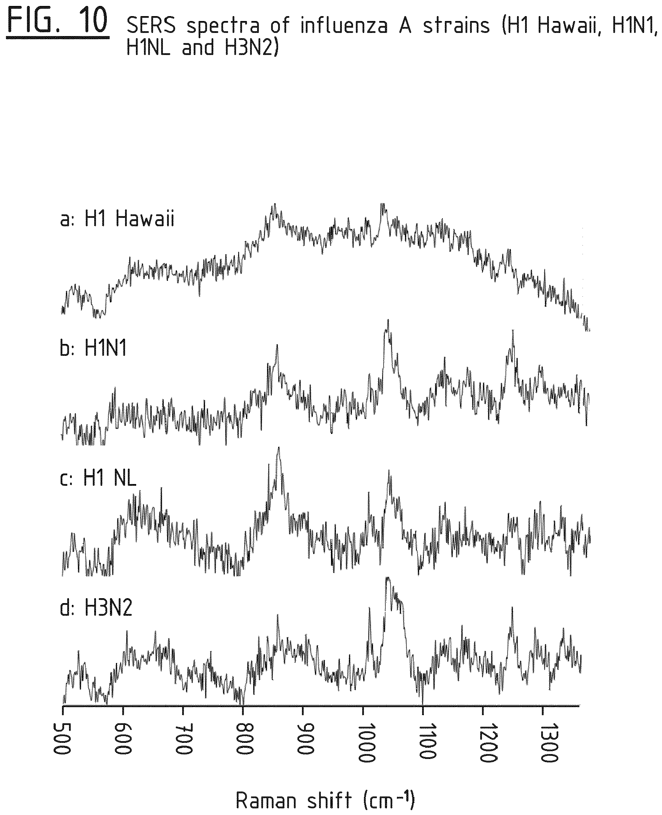

Figure 10 SERS spectra for inclusion in a database as provided herein of influenza A strains (H1 Hawaii, H1N1, H1NL and H3N2) are shown. Influenza virus strains were shown to present differences in the SERS spectra specifically to their type. The spectra of these virus strains have distinct characteristic Raman peak shift assignments as shown in table 3.

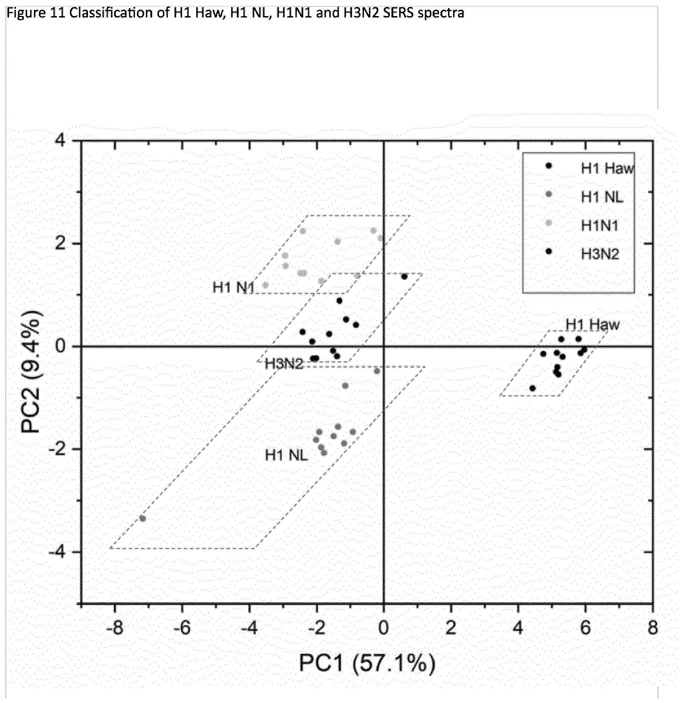

Figure 11 Classification of H1 Haw, H1 NL, H1N1 and H3N2 SERS spectra by PCA is shown. We further investigated the feasibility of using SERS using a chip according to the invention to differentiate four different influenza virus strains, PCA was conducted based on SERS spectra acquired from 4 Influenza virus samples (H1 Haw, H1 NL, H1N1 and H3N2) on range between 400-1360 cm⁻¹(Figure 10). For the PCA model a total of 44 spectra (by determination of 11 replicants per each virus strain) were used and a two-dimensional score plot was created using the principle component 1 vs principal component 2. The score plots demonstrate the clustering of four different virus strains.

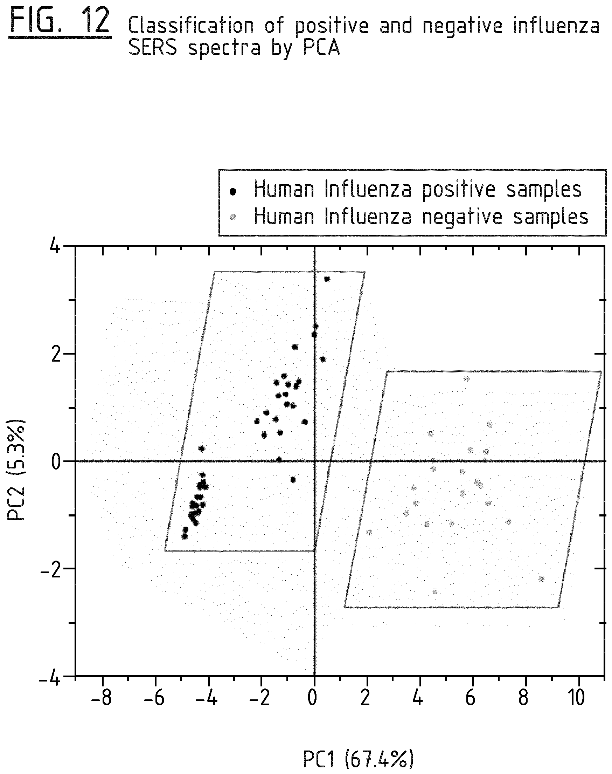

Figure 12 Classification of positive and negative Influenza SERS spectra by PCA is shown. All tested positive influenza samples tested positive, all tested negative influenza samples tested negative, indicating that close to 100% sensitivity as well as close to 100% specificity may be obtained in testing influenza virus in Raman detection with a chip according to the invention.

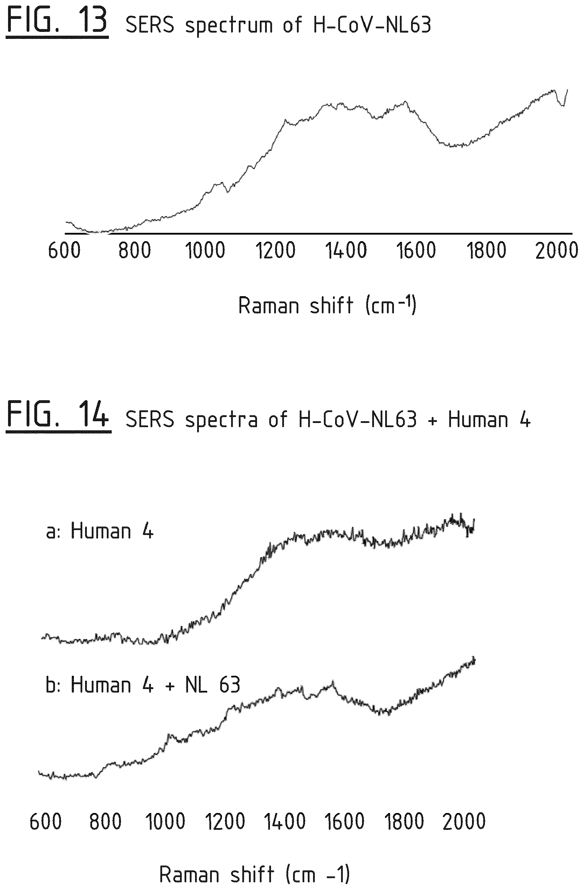

Figure 13 SERS spectrum for inclusion in a database as provided herein of H-CoV-NL63. In table 4 the Raman shifts of the peaks of the Raman spectrum are given. These peaks are assigned to their corresponding chemical bands. H-CoV-NL63 shows characteristic peaks which can be related to specific components of the virus.
Figure 14 SERS spectra for inclusion in a database as provided herein of H-CoV-NL63 + human 4. Raman spectra of the H-Cov_NL63 spiked human sample have similar peak assignment as the H-Cov-NL63 Raman spectra, as tested alone, therefore it can be said that a human negative sample does not hold back viral signals.

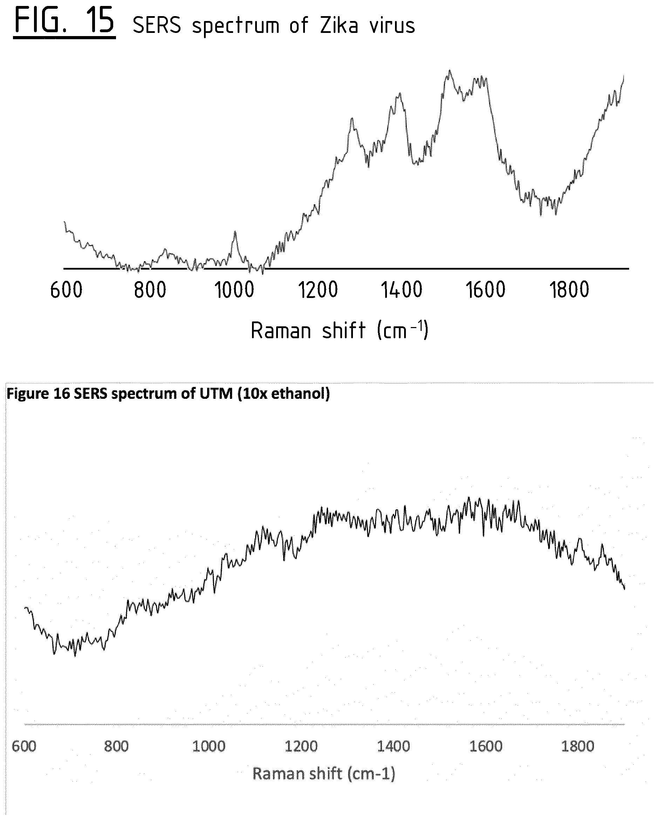

Figure 15 SERS spectrum for inclusion in a database as provided herein of Zika virus. In table 5 the Raman shifts of the peaks of the Raman spectrum are given. These peaks are assigned to their corresponding chemical bands. *Zika* shows characteristic peaks which can be related to specific components of the virus.
Figure 16 SERS spectrum for inclusion in a database as provided herein of UTM (10x ethanol)

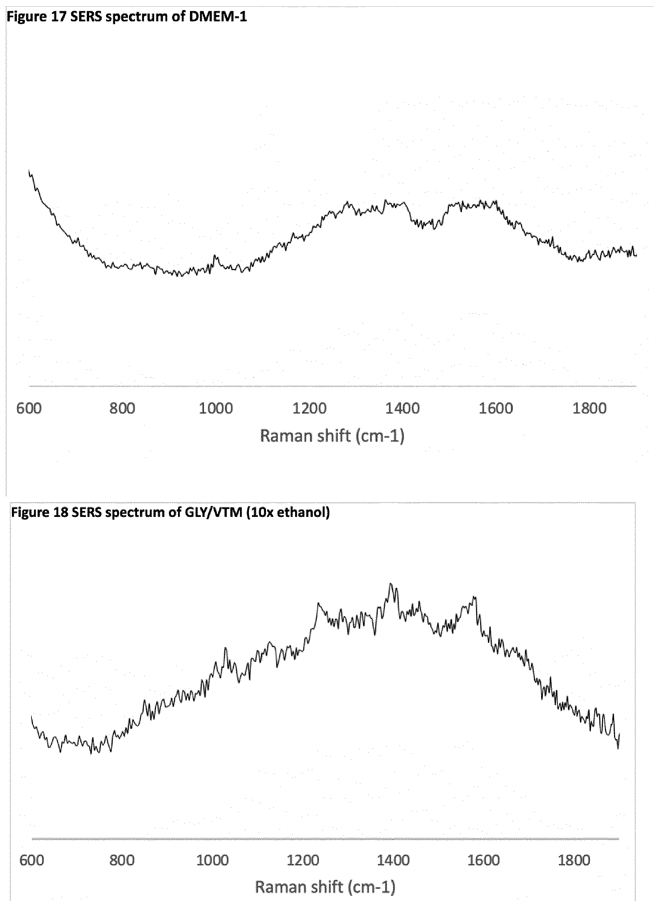

Figure 17 SERS spectrum for inclusion in a database as provided herein of DMEM-1
Figure 18 SERS spectrum for inclusion in a database as provided herein of GLY/VTM (10x ethanol)

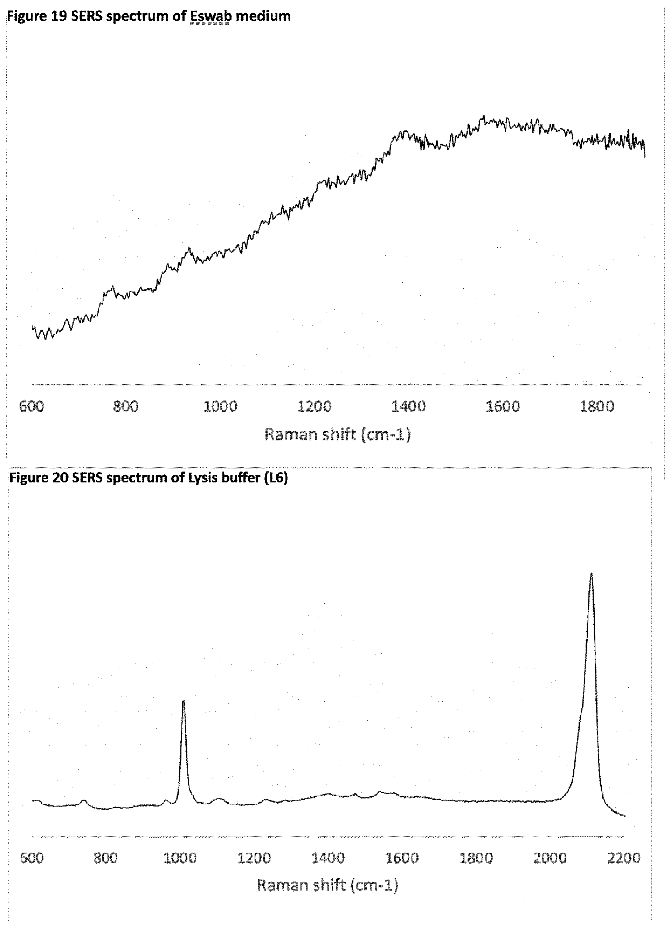

Figure 19 SERS spectrum for inclusion in a database as provided herein of Eswab medium
Figure 20 SERS spectrum for inclusion in a database as provided herein of Lysis buffer (L6).
   As is clear from the spectra as shown in the figures 16-20, various media contain Raman active molecules. The spectra of these media can potentially hide Raman signals from Raman signals of the virus molecules. By identifying which peaks are from the media, peaks can better be attributed that are specific for the virus. Some media show noisier spectra than others.

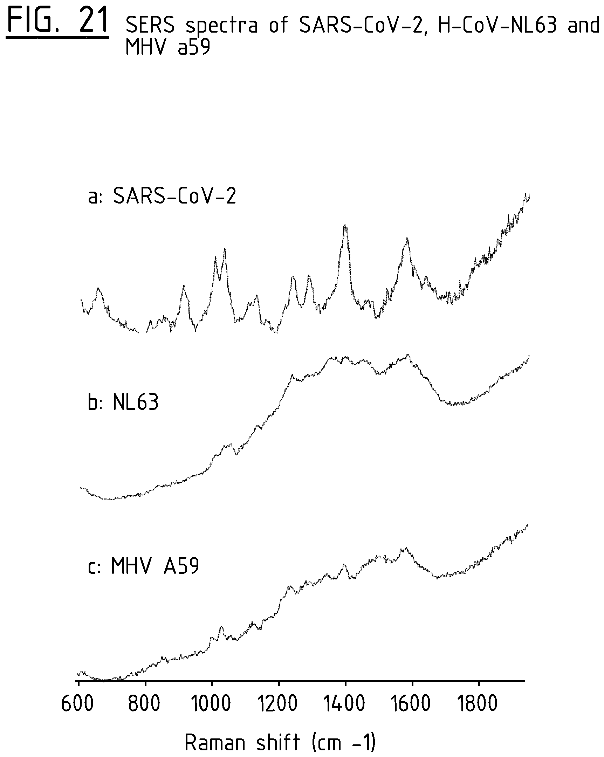

Figure 21 SERS spectra for inclusion in a database as provided herein of SARS-CoV-2, H-CoV-NL63 and MHV a59. In table 6 the Raman shifts of the peaks of the Raman spectrum, showed in figure 21a, are given. These peaks are assigned to their corresponding chemical bands. SARS-CoV-2 show characteristic peaks, the peaks 650 cm⁻¹ (Guanine (3)), 814 cm⁻¹ (one of the most distinct peaks of RNA), 867 cm⁻¹ (ribose vibration of RNA), 915 cm⁻¹ (ribose vibration of RNA), 974 cm⁻¹ (ribose vibration of RNA), 1244 cm⁻¹ (one of the most distinct peaks of RNA), 1285 cm⁻¹ (cytosine), and 1506 cm⁻¹ (N=H bending of cytosine) are peaks related to RNA. The peak at 850 cm⁻¹ is due to C-C stretch of the amino acid tyrosine. The peaks at 1007 cm⁻¹ (the in-plane C-H bending mode of phenylalanine) and 1030 cm⁻¹ (symmetric ring breathing mode of phenylalanine (3,4)) and 1216 cm⁻¹ (C-N and C-C₆H₅ stretching) and 1586 cm⁻¹ (C=C bending mode of phenylalanine) are related to the amino acid phenylalanine. The peak at 1400 cm⁻¹ is from the COO- stretching of protein amino acids close to the nanoparticles surface. The peak at 1448 cm⁻¹ is due to the CH deformation of phenyl groups. The peak at 1461 cm⁻¹ is the CH₂ deformation of lipids which indicates the lipid envelope.

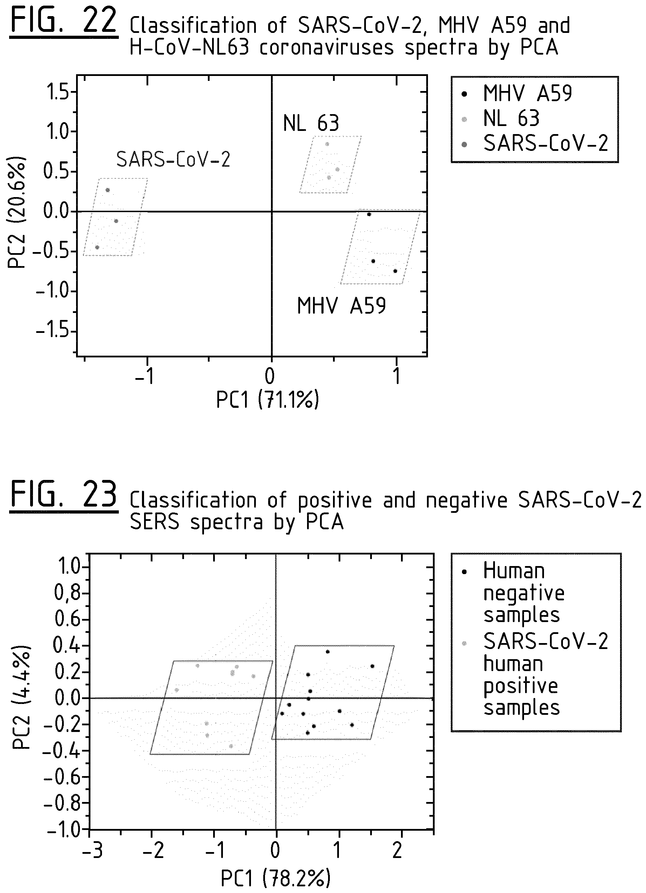

Figure 22 Classification of SARS-CoV-2, MHV A59 and H-CoV-NL63 coronaviruses spectra by PCA. For the PCA model a total of 9 spectra (by 3 replicants per each virus sample) were used and a two-dimensional score plot was created using the principle component 1 vs principal component 2. The score plots demonstrate the clustering of three different coronaviruses. More specifically, a positive value for PC1 suggests the sample is likely to be MHV A59 while a negative score for PC 1 suggests the sample contains SARS-CoV-2. Similarly, a positive value for PC 2 indicates that the sample is likely to be H-CoV-NL63. The combination of the information contained in PC1 and PC2 axes reveals a clear discrimination between different types of coronaviruses. These results show that the invention as provided herein can be used to differentiate between viruses of the same family type such as coronaviruses.
Figure 23 Classification of positive and negative SARS-CoV-2 SERS spectra by PCA, to make an effective classification of SARS-CoV-2 positive and negative samples, PCA was also used to establish significant differences between these SERS spectra. The PCA model generated was used to classify the 21 spectra (3 SARS-CoV-2 positive samples and 4 negative samples by 3 replicates each) in a range between 800-1700 cm⁻¹. The samples were diluted in EtOH and then centrifuged (raster on). A two-dimensional score plot that was created using the principle component 1 vs principal component 2. The score plots demonstrate a clear clustering between SARS-CoV-2 human positive and negative specimens, obtaining 100% specificity in detecting known true negative samples and 100% sensitivity in detecting known true positive samples. More specifically, a positive value for PC1 suggests the sample is likely to be negative while a negative score for PC 1 suggests the samples are SARS-CoV-2 positive. The combination of the information contained in PC1 and PC2 axes reveals a clear discrimination between positive and negative. These results are direct evidence that a method according to the invention as provided herein can be employed as SARS-CoV-2 detection tool and the discrimination of virus-negative subjects from virus-positive subjects.

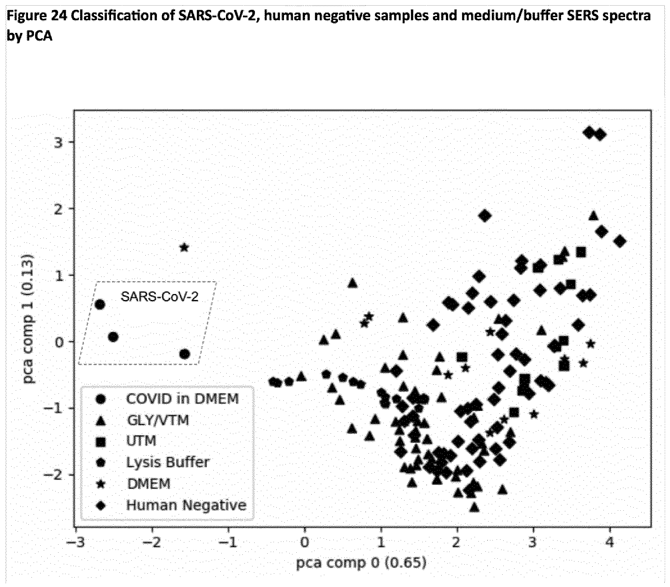

Figure 24 Classification of SARS-CoV-2, human negative samples spectra, and medium/buffer SERS spectra by PCA. To further demonstrate close to 100% specificity, negative human swab samples were tested, together with samples of media and buffers commonly used, (a total of 670 negative samples were tested) in relation to SARS-Cov-2 positive signature samples. None of the known negative samples tested positive.
Figure 25 Constructed barcodes for inclusion in a database as provided herein of Raman spectrum of SARS-CoV-2

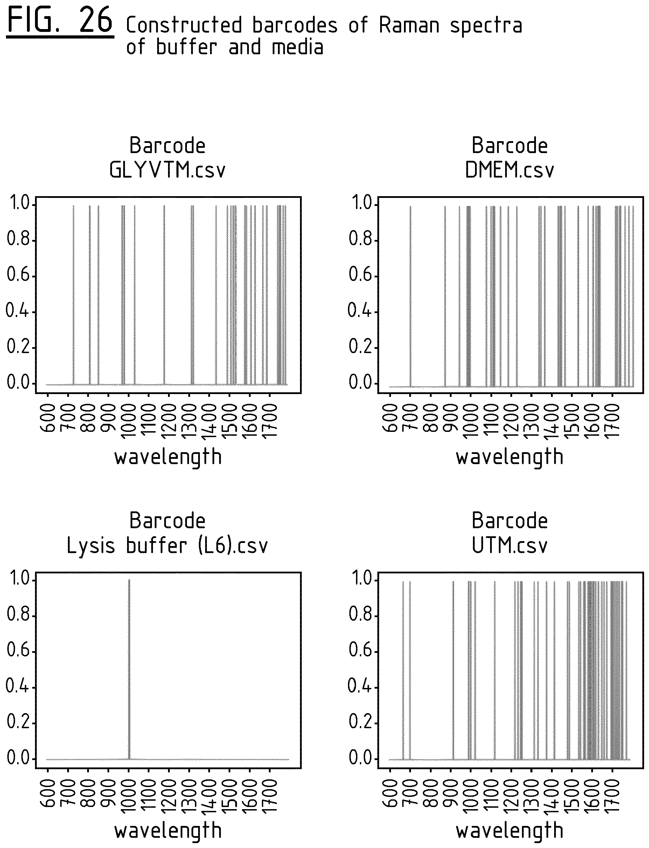

Figure 26 Constructed barcodes for inclusion in a database as provided herein of Raman spectra of buffer and media biomolecules present in a sample

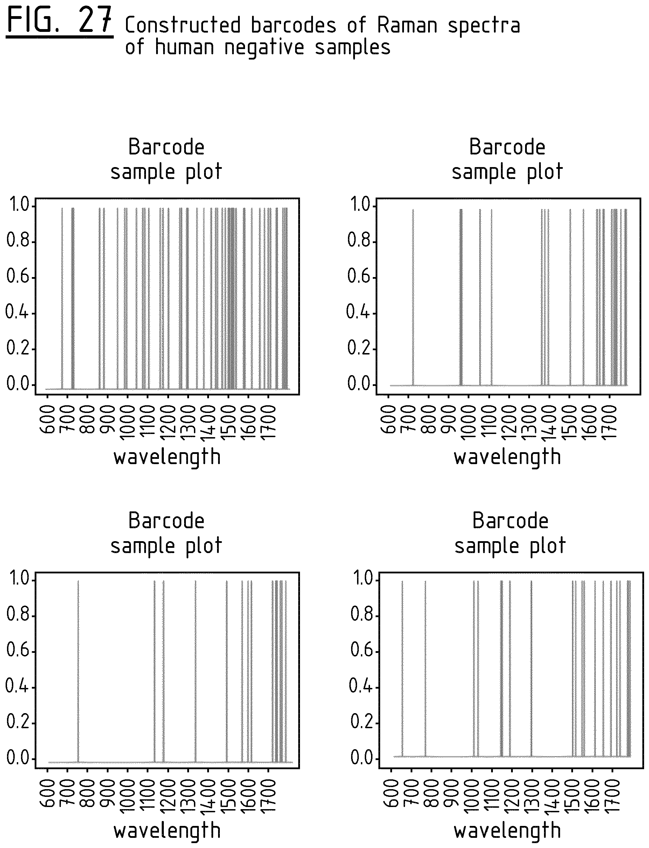

Figure 27 Constructed barcodes for inclusion in a database as provided herein of Raman spectra of human negative samples

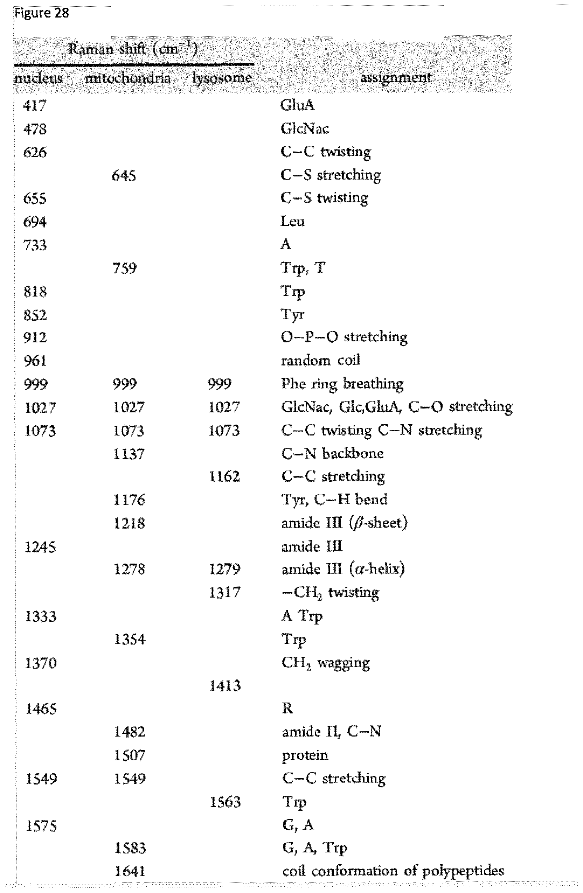

Figure 28 SERS peak shift assignments of biomolecules in human cellular organelles for inclusion in a database as provided herein. Shen et al, Organelle-targeting surface-enhanced Raman scattering (SERS) nanosensors for subcellular pH sensing. Nanoscale, 2018,10, 1622-1630

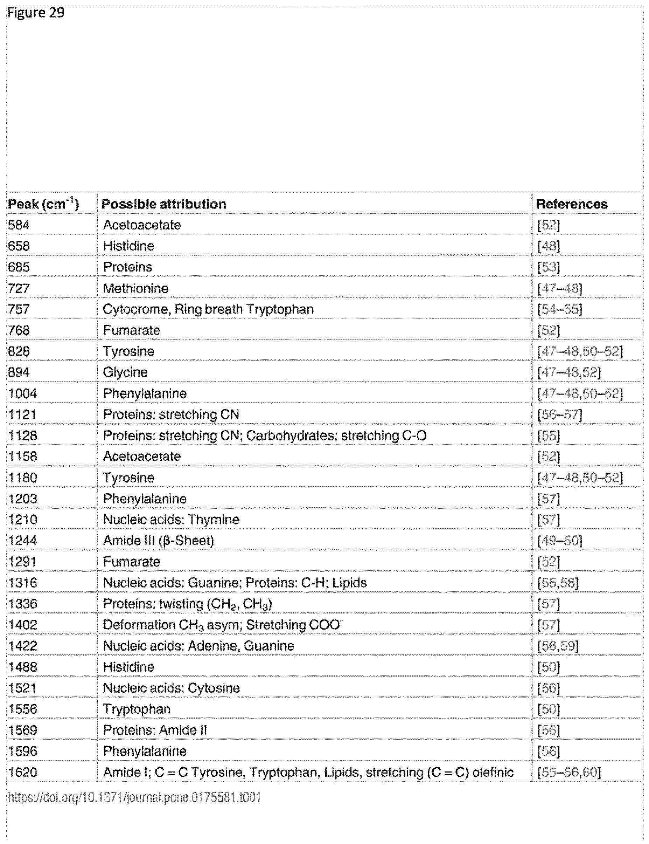

Figure 29 SERS peak shift assignments of biomolecules extracted from cells for inclusion in a database as provided herein. Shalabaeva V, Lovato L, La Rocca R, Messina GC, Dipalo M, et al. (2017) Time resolved and label free monitoring of extracellular metabolites by surface enhanced Raman spectroscopy. PLOS ONE 12(4): e0175581. https://doi.org/10.1371/journal.pone.0175581

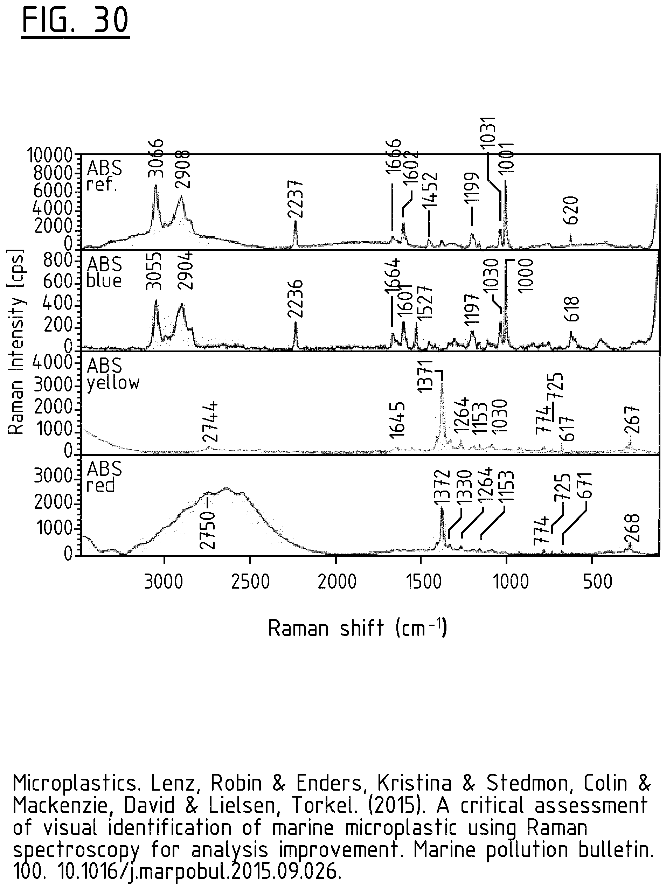

Figure 30 SERS peak shift assignments of biomolecules for microplastics. Lenz, Robin & Enders, Kristina & Stedmon, Colin & Mackenzie, David & Nielsen, Torkel. (2015). A critical assessment of visual identification of marine microplastic using Raman spectroscopy for analysis improvement. Marine pollution bulletin. 100. 10.1016/j.marpolbul.2015.09.026.

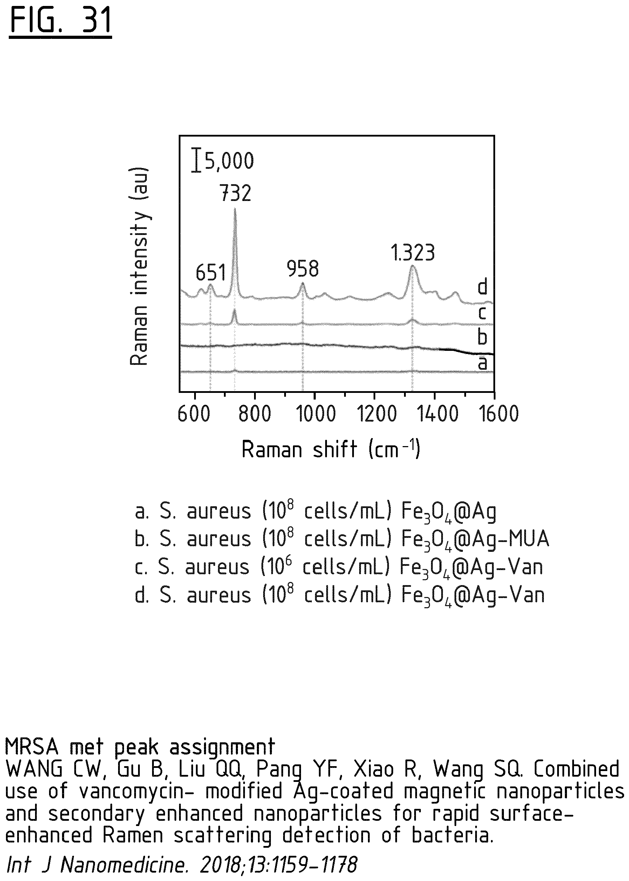

Figure 31 SERS peak shift assignments of biomolecules for inclusion in a database as provided herein for MRSA. Wang CW, Gu B, Liu QQ, Pang YF, Xiao R, Wang SQ. Combined use of vancomycin-modified Ag-coated magnetic nanoparticles and secondary enhanced nanoparticles for rapid surface-enhanced Raman scattering detection of bacteria. Int J Nanomedicine. 2018;13:1159-1178

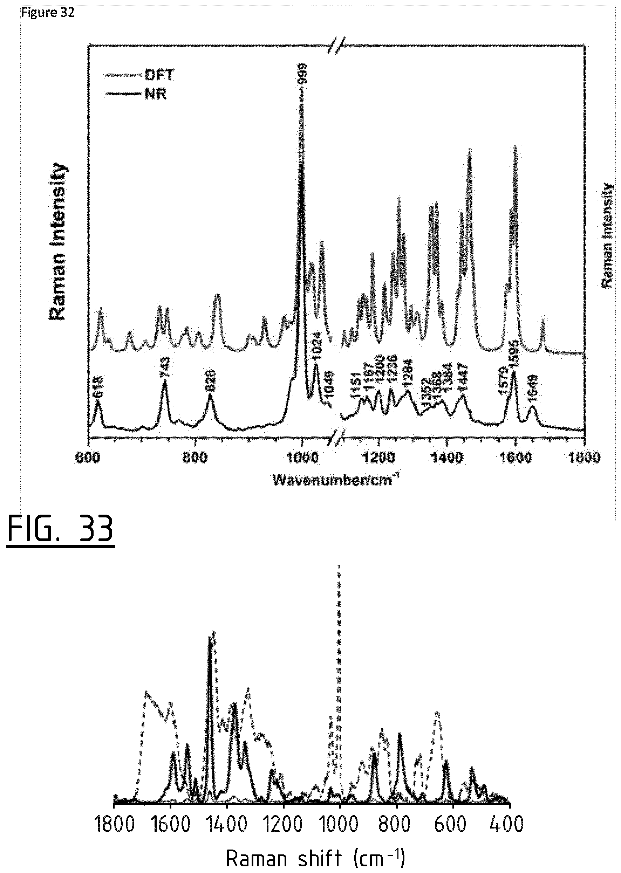

Figure 32 SERS peak shift assignments for inclusion in a database as provided herein for Fentanyl Leonard, Jennifer & Haddad, Abed & Green, Omar & Birke, Ronald & Kubic, Thomas & Kocak, Ali & Lombardi, John. (2017). SERS, Raman, and DFT analyses of fentanyl and carfentanil: Toward detection of trace samples. Journal of Raman Spectroscopy. 48. 10.1002/jrs.5220.
Figure 33 SERS peak shift assignments of biomolecules for inclusion in a database as provided herein for mycoplasma. Differentiation of Mycoplasma spiked and control throat swab samples.
   Representative difference spectra of spiked, pooled throat swab samples after subtraction of the spectrum of an un-spiked, pooled throat swab control. Bold and thin solid lines, spiked samples (8.2x104 and 8.2x103 CFU M. pneumoniae M129/µl, respectively); dashed line, representative spectrum of M. pneumoniae M129.

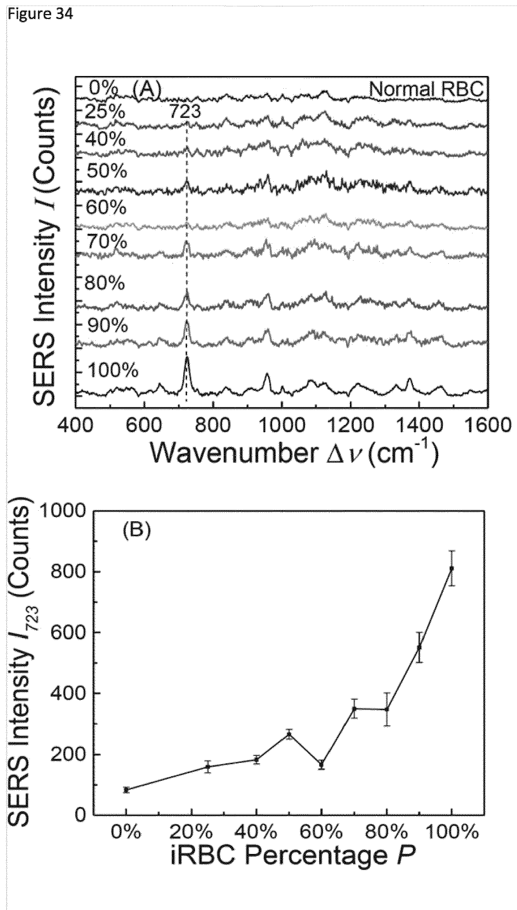

Figure 34 SERS peak shift assignments of biomolecules for inclusion in a database as provided herein for malaria. SERS spectra of mixtures of red blood cells (RBCs) and malaria schizont stage infected red blood cells (iRBCs) at a fixed cell concentration of 3×10⁸/ml but with different mixing percentage P; and (B) the plot of SERS peak intensity *I*₇₂₃ of the mixtures versus the iRBC percentage P.

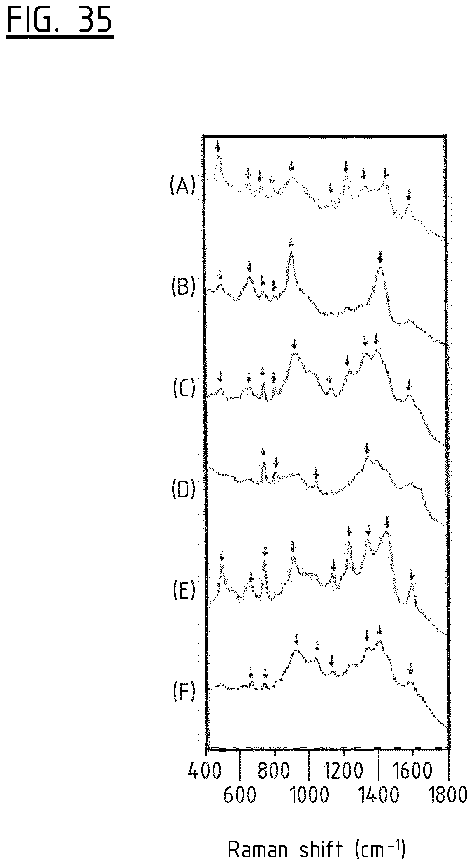

Figure 35 SERS peak shift assignments of biomolecules for inclusion in a database as provided herein for mycelia of the fungal pathogens collected from banana.
   The Raman spectra databases of the fungal pathogens on banana in Taiwan were built, including databases for (A) *Botryosphaeria dothidea* (which causes Crown rot of banana, YJC-F004), (B) *Deightoniella torulosa* (which causes Deightoniella leaf spot, PM-YJL-F119), (C) *Alternaria alternata* (which causes Alternaria speckle of banana, PM-TYC-F003), (D) *Cordana musae* (which causes Cordana leaf spot of banana, LNH-F001), (E) *Colletotrichum musae* (which causes Anthracnose of banana, PM-YHL-F001), and (F) *Fusarium oxysporum* f. sp. *cubense* tropical race 4 (which causes Fusarium wilt of banana, PM-TYC-F040). Average Raman spectra were obtained from five independent replications of the surface-enhanced Raman spectroscopy measurements. https://doi.org/10.1371/journal.pone.0230330.g001
Figure 36 SERS peak shift assignments of biomolecules for inclusion in a database as provided herein for Malaria. Hamm, Logan & Gee, Amira & Indrasekara, A.. (2019). Recent Advancement in the Surface-Enhanced Raman Spectroscopy-Based Biosensors for Infectious Disease Diagnosis. Applied Sciences. 9. 1448. 10.3390/app9071448.
Figure 37 SERS peak shift assignments of biomolecules for inclusion in a database as provided herein for Estrogen Liu, Y., Chen, Y., Zhang, Y., Kou, Q., Zhang, Y., Wang, Y., Chen, L., Sun, Y., Zhang, H., & MeeJung, Y. (2018). Detection and Identification of Estrogen Based on Surface-Enhanced Resonance Raman Scattering (SERRS). Molecules (Basel, Switzerland), 23(6), 1330.

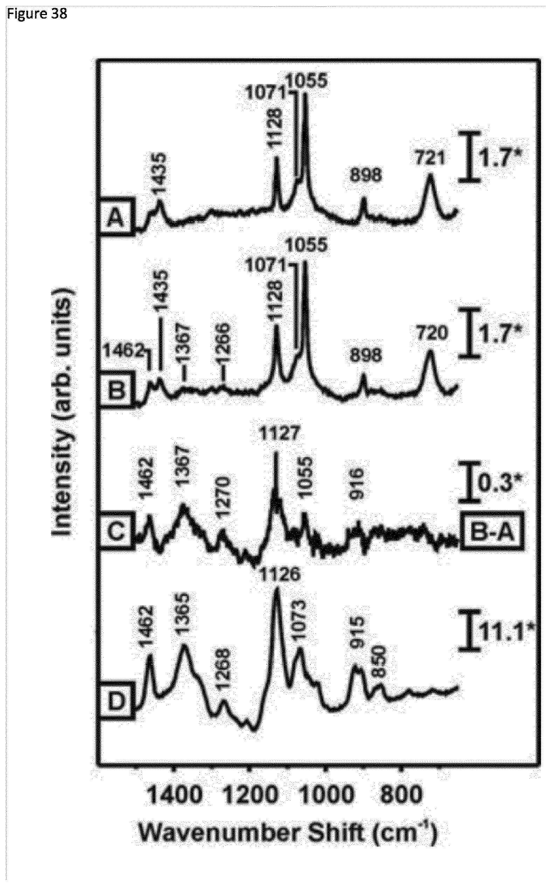

Figure 38 SERS peak shift assignments of biomolecules for inclusion in a database as provided herein for Glucose Yonzon, Chanda & Lyandres, Olga & Shah, Nilam & Dieringer, Jon & Duyne, Richard. (2006). Glucose Sensing with Surface-Enhanced Raman Spectroscopy. 10.1007/3-540-33567-6_19.

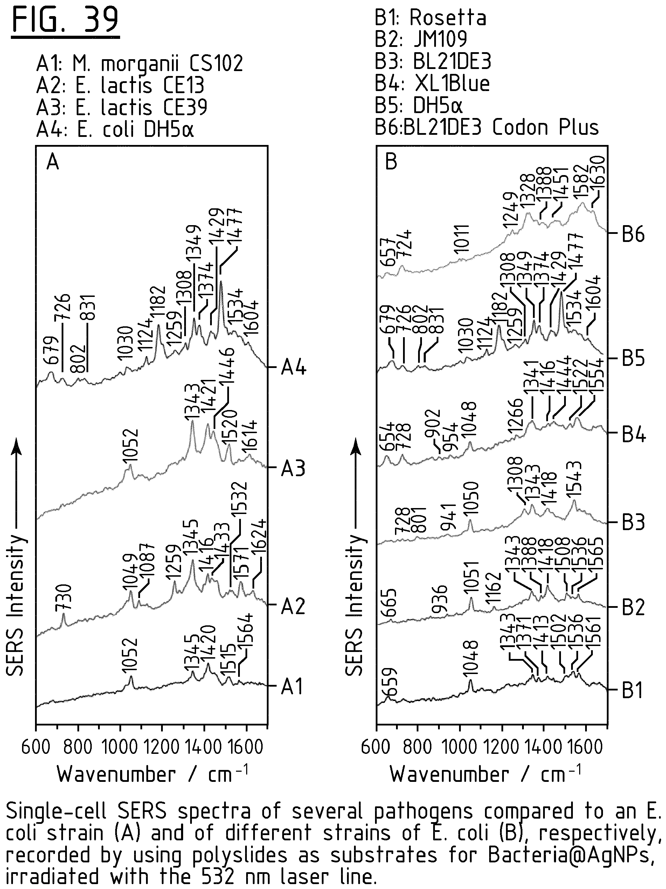

Figure 39 SERS peak shift assignments of biomolecules for inclusion in a database as provided herein for Pesticide Fan, Nancy & Lai, Keqiang & Rasco, Barbara & Huang, Yiqun. (2015). Determination of carbaryl pesticide in Fuji apples using surface-enhanced Raman spectroscopy coupled with multivariate analysis. LWT - Food Science and Technology. 60. 352-357. 10.1016/j.lwt.2014.08.011.

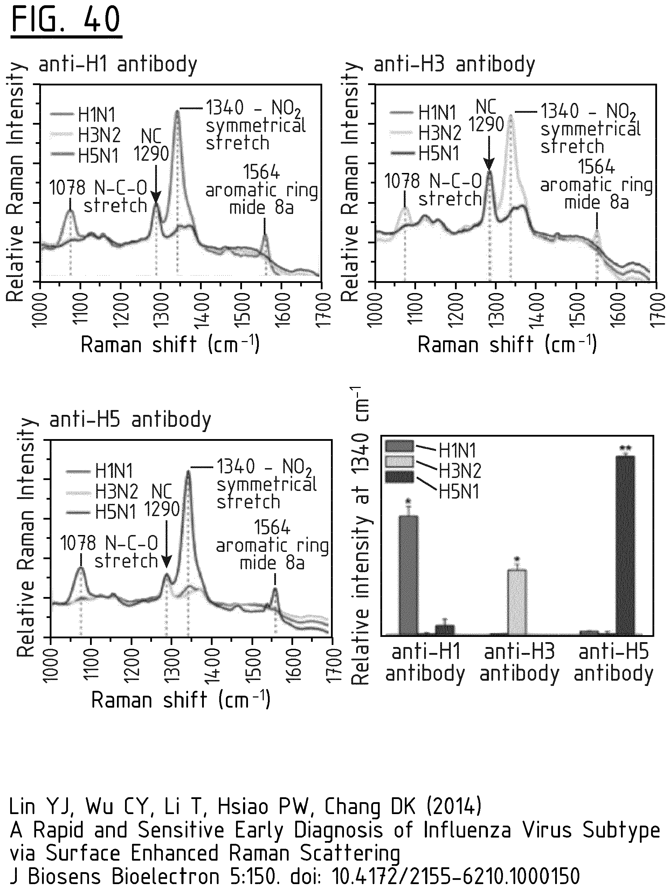

Figure 40 SERS peak shift assignments of biomolecules for inclusion in a database as provided herein for Antibodies Lin, Y.J. et al. (2014) A Rapid and Sensitive Early Diagnosis of Influenza Virus Subtype via Surface Enhanced Raman Scattering.

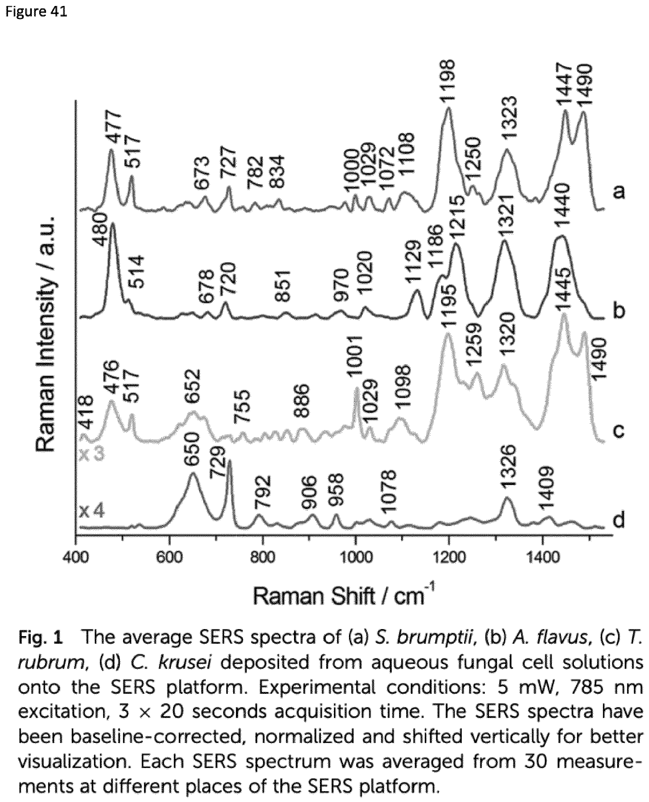

Figure 41 SERS peak shift assignments of biomolecules or inclusion in a database as provided herein for Fungal Pathogens (a.o. candida) Witkowska, Evelin & Jagielski, Tomasz & Kamińska, Agnieszka & Kowalska, Aneta & Hryncewicz-Gwóźdźd, Anita & Waluk, J.. (2016). "Detection and identification of human fungal pathogens using surface-enhanced Raman spectroscopy and principal component analysis". Anal. Methods. 8. 10.1039/C6AY02957D.

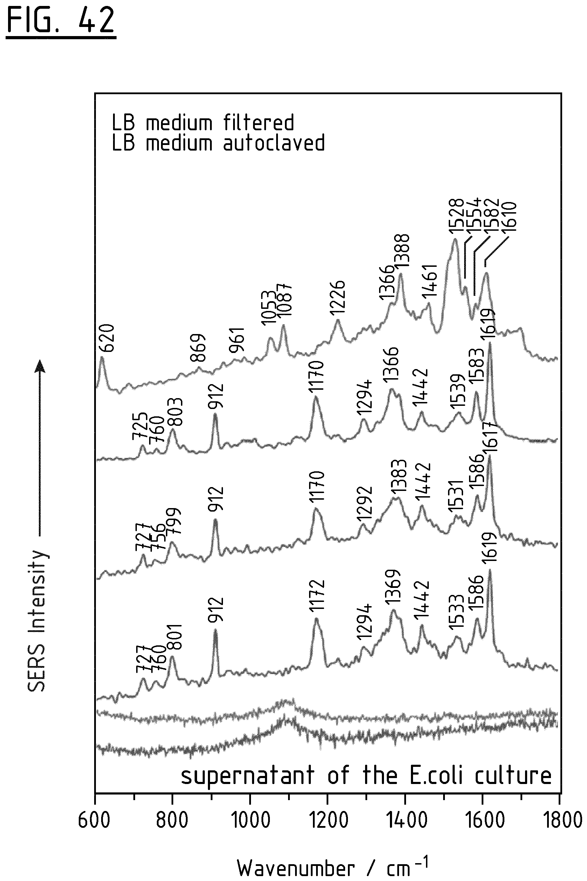

Figure 42 SERS peak shift assignments of biomolecules for inclusion in a database as provided herein for Bacterial Pathogens Mircescu, Nicoleta & Zhou, Haibo & Colnita (Ungurean), Alia & Leopold, Nicolae & Nagy-Szoke, Tiberius & Coman, Cristian & Haisch, Christoph. (2017). Rapid single-cell detection and identification of pathogens by using surface-enhanced Raman spectroscopy.

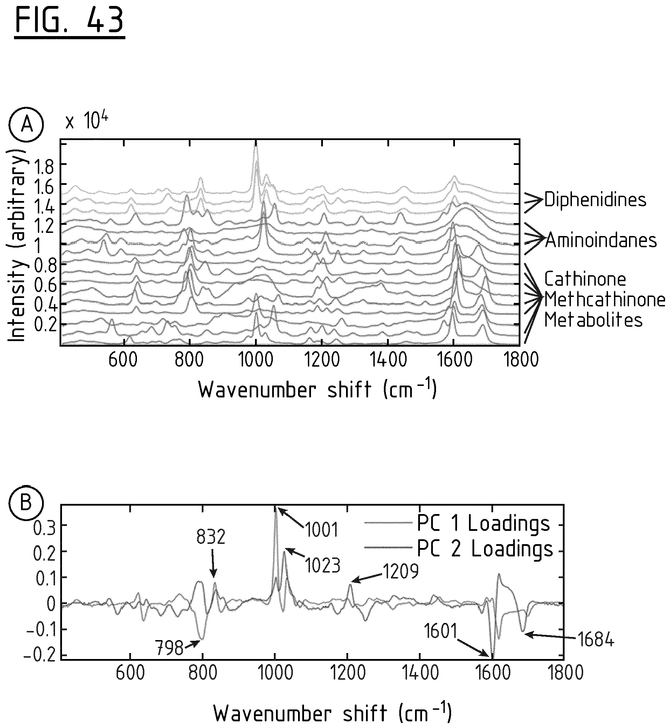

Figure 43 SERS peak shift assignments of biomolecules for inclusion in a database as provided herein for psychoactive substances. Muhamadali et al., Front. Chem., 19 June 2019 | https://doi.org/10.3389/fchem.2019.00412

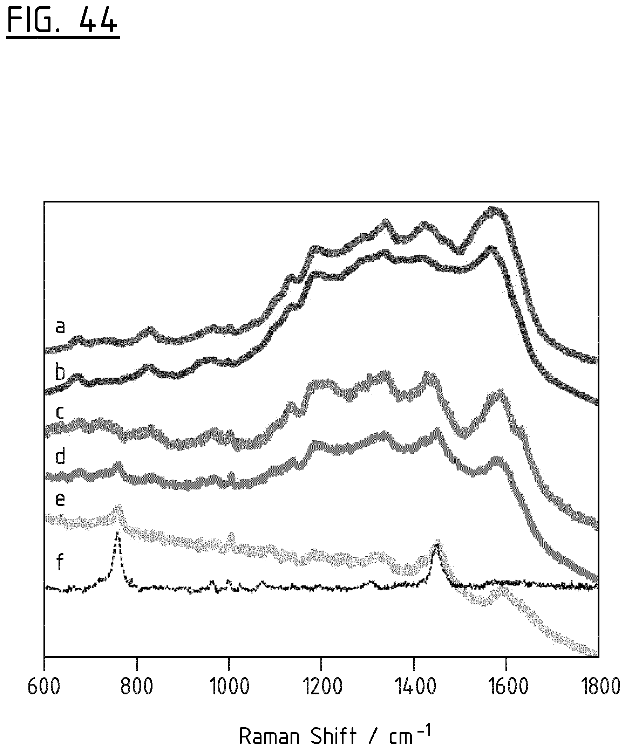

Figure 44 SERS peak shift assignments of biomolecules for inclusion in a database as provided herein for prion. Alvarez-Puebla et al, Proc Natl Acad Sci USA. 2011 May 17; 108(20): 8157-8161.

### Detailed description

Biomolecule detection in surface enhanced Raman spectroscopy.

Less than forty years have passed since the initial discovery of strong Raman signals enhanced at a rough silver surface, which quickly gave rise to a new emerging research field - surface enhanced Raman spectroscopy, or in brief: SERS. SERS enhancement occurs during the interaction of an incident electromagnetic field with surface plasmon resonance at a metal surface. High SERS enhancement factors (>10⁷-10⁸) can be developed at the interparticle junctions of plasmonic nanostructures ('plasmonic hot spots'), sufficient enough to enable biomolecule detection.

Raman spectroscopy is a type of vibrational spectroscopy that can measure how the radiation is scattered by the material. With this highly sensitive technique, complex molecular structures and details can be acquired. Raman spectroscopy needs only a minimal amount of sample, but it has a major disadvantage since the Raman scattering effect is very weak. Though Raman scattering can provide very useful information, it produces low signal intensity. There was a great discovery, reported in a published paper in 1974, of a remarkable enhancement of the Raman scattering effect from pyridine molecules adsorbed onto a silver electrode comparing to free molecules in a liquid environment. This discovery eventually led to Surface Enhanced Raman Scattering (SERS), which is a technique that enhances the Raman scattering intensity originating from molecules located in the vicinity of nanostructured metallic surfaces. Hence, the improvement of the Raman scattering intensity is accomplished by absorbing the sample onto a metal substrate which helps amplify electric fields of the incident and scattered light. The mechanism of this SERS effect is not yet fully understood, but there are two theories that suggest the effect could relate to a chemical or electromagnetic enhancement. The SERS enhancement value can be as high as 10¹⁰ and can therefore be considered a promising single molecule analysis tool. The metals used for SERS substrates can be selected by the plasmon resonance frequency. As mentioned above, molecular vibrations can be measured, and the measurements of detected vibrations can shine light onto molecular structures. Instrumentation to detect these vibrations is well developed in near-infrared and the visible Raman regions of the spectrum. The plasmon resonance frequencies of silver, gold and copper (Ag, Au and Cu) are in these regions and therefore they tend to produce the strongest enhancement when used as SERS substrates.

SERS entirely depends on the strongest plasmon resonances on the surface, either with a relatively uniform enhancement or with large variations. The latter, i.e. plasmonic hot spots, enable single molecule detection. However, strong enhancement is not the only requirement for the SERS detection of individual molecules, and the surface area needs to be considered as well. As SERS is a surface spectroscopy tool, a larger surface area with a periodic array of plasmonic hot spots can increase the detection of a greater potential number of individual molecules. Direct contact of the analyte with plasmonic hot spots in the first surface layers is another SERS requirement. A typical single molecule SERS spectrum appears as signals randomly fluctuating with time at local positions with the enhancement on the SERS substrate. For this reason, the single molecule SERS phenomenon is controversial, and indeed there has been much debate about its existence and application over the years.

As single molecule SERS is a highly local effect. Understanding of the various modes of its coupling into the plasmon modes at hot spots is important for the development of new plasmonic nanostructures and theoretical models for SERS emission (Weber et al., , ACS Nano, 2012, 6 , 1839). How the nanoparticle geometry, dimensions and sustainability contribute to the electromagnetic field enhancement at hot spots in the SERS phenomenon is not yet fully understood. What is the diffusion behaviour of analytes at the plasmonic hot spots? (Willets and Stranahan , Proc. SPIE, 2012, 8228, 82280P). From a fundamental point of view, it is important to understand how the average enhancement factor can contribute to each molecule under SERS conditions on the surface. In this aspect, one of the critical parameters is an analyte adsorption and positioning at the plasmonic hot spot, especially concerning the transfer of an analyte from the bulk to the SERS active surface. The adsorption efficiency directly affects the analytical SERS enhancement factors. For example, 10% adsorption efficiency means 10 times less signal at a given concentration.

One molecule positioned at plasmonic hot spot can provide 10 times higher signal than one only a few nanometers away and 100 times more than SERS signal from randomly adsorbed molecules. Le Ru and Etchegoin, MRS Bull., 2013, 38, 631. Hot spot formation on the surface of substrates for use in SERS is often a highly local and unevenly distributed effect occurring only at sharp edges, interparticle junctions and crevices or other geometries with a sharp nano roughness of plasmonic nanostructures. The spectral emission of a biomolecule at SERS conditions depends on the local enhancement field of the hot spots, the evenness of distribution of hot spots, its frequency of occurrence, as well as the binding affinity and positioning of the analyte at a hot spot region. Furthermore, the stability of near-field nano-optics at hot spots is deemed critical, particularly in a biological milieu. Radziuk and Moehwald, Phys. Chem. Chem. Phys., 2015,17, 21072.

Hot spot availability is generally taught improved through improvement of geometrical structures of nanosurfaces.

Various methods have been developed to improve SERS conditions needed to for molecule detection. These improvements are generally aimed to improve position of analytes at geometrically structured hot spots regions, considered to result in improvements in analytical enhancement factors and in the statistics of molecule detection. Various colloidal lithography methods, self-assembly procedures and surface-functionalization chemistry techniques relating to regular and geometrically conserved nanoparticle structures have been advanced in this regard. However, the transportation of analytes from the bulk solution to the SERS active surface, selective positioning at hot spots, and overall hot spot availability to have improved enhancement, is still a critical problem especially in a biological environment.

The main task of SERS is the amplification of weak Raman signals. In this regard, electromagnetic field enhancement is a dominating factor that in general is thought to be well controlled if one governs the relationship between the localized surface plasmon effect and the metallic or metallic/dielectric nanostructure, i.e. the pre-arranged physico-chemical structural properties of the plasmonic hot spots.

Plasmonic hot spots with a great variety of geometries have been produced ever since the first prediction of higher values of the electromagnetic field increasing at the sharp edges of nanostructures (Marinica et al, Nano Lett., 2012, 12, 1333). Song-Yuan Ding (Chem. Soc. Rev., 2017,46, 4042-4076) poses that surface-enhanced Raman spectroscopy (SERS) and related spectroscopies are powered primarily by the concentration of the electromagnetic (EM) fields associated with light in or near appropriately nanostructured electrically-conducting materials, most prominently, but not exclusively in high-conductivity metals such as silver and gold. Well known Klarite® substrates, for example, are considered to act enhancing through an Au surface carrying inverted pyramids with 2000 nm pitch length and 1000 nm pit depth, with square cross section. Pilat et al., (Sensors (Basel). 2018 Oct; 18(10): 3212) further develop the Klarite structured concept and employ a SERS substrate named SK307 based on a silicon chip, on which structures are fabricated using electron beam lithography and wet etching to achieve a pattern of inverted pyramids on the surface, which was then covered by gold layer of defined thickness and roughness. Also, silver nanoparticles with a triangular, rectangular or oval shape exhibit relative strong electromagnetic field enhancement contours, with enhancement factors reaching up to 103 (Hao and Schatz, J. Chem. Phys., 2004, 120, 357). In contrast, the electromagnetic field increase is considered at least one order of magnitude less efficient when using spherical silver nanoparticles (Radziuk and Moehwald, Phys. Chem. Chem. Phys., 2015,17, 21072).

There have been attempts over the past time since the SERS discovery in improving the SERS technology and working on the limitations and improving the requirements. To get rid of some bottlenecks other concepts have arisen, such as tip-enhanced Raman spectroscopy (TERS) and shell-Isolated nanoparticle-enhanced Raman spectroscopy (SHINERS). TERS technology has been developed since the year of 1985 and is a technique that is similar to SERS only the enhancement of the Raman signal happens at the point of the sharp tip that is usually coated with a metal, gold or silver nanoparticles. This newer technology overcomes some limitations of SERS by providing noncontact detection of target molecules on every type of surface. Therefore, the metal tip and the sample of target molecules do not have to interact with each other. Though, some other problems come along with the TERS technology, such as the Raman enhancement in TERS is very narrow as the enhancement is between only the tip and a few molecules and therefore problems in sensitivity can arise. Another technique based on enhancement in Raman spectroscopy that arose in 2010, is SHINERS. This technology has shell-isolated nanoparticles distributed over a surface, which can be any surface and therefore this technology has been very attractive, and these nanoparticles tend to behave like small TERS tips. This technique overcomes contamination and agglomeration problems etc. of the nanoparticles. Though the enhancement of the Raman signal in this technology is lower in comparison to what the SERS technology provides.

Improved hot spot availability is obtainable through sintering with spark ablation and impaction resulting in fractal-patterned multi-creviced sintered agglomerates of metal-based nanoparticles.

The desired surfaces of this invention are therewith generated through a diffusion-limited aggregation processes of said metal nanoparticles or compositions and compounds thereof, during deposition and sintering of said nanoparticles on said surface. This method of deposition or printing nanoparticles creates the ultimately desired rough surface coverage with desired surface hot spot morphology in on a layer of nanoparticles. Through deposition by impaction, nanoparticles tend to aggregate into randomly sintered nanoparticles in a layer of fractal-patterned multi-creviced sintered agglomerates of metal nanoparticles or agglomerates of metal composition or agglomerates of metal compound nanoparticles, as can be observed in the TEM picture in figure 2.2 and SEM picture in figure 4b. Increasing the speed of the gas during particle production results in smaller particles (reducing the time of flight/residence time). Lowering the vacuum in the deposition chamber may result in a higher impaction speed, and a denser layer with the particles more closely together). A typically useful nanoparticle agglomerate for use in the invention as provided herein may be produced using a nanoparticle generator such as VSP-G1 nanoparticle generator combined with the VSP-P1 nanostructured materials printer, commercially available from the company VSParticle (VSPARTICLE B.V. Molengraaffsingel 10, 2629 JD Delft, The Netherlands, info@vsparticle.com), wherein the impaction conditions may be controlled.

In particularly impaction speed influences locally increased temperatures of nanoparticle material upon impact to the surface that contributes to the, at least partial, liquefaction of said particles upon impact, thus contributing to the sintering process that results in deposition of fractal-patterned multi-creviced sintered agglomerates of metal nanoparticles or agglomerates of metal composition or agglomerates of metal compound nanoparticles on the targeted surface. As the chip surface temperature itself is generally held at room temperature, or at least sufficiently below the melting point of the metal nanoparticle material in question, the liquefaction and sintering process rapidly stops after impact and deposition on the chip, acutely arresting the sintering process, therewith shock-freezing the agglomerates in the desired fractal-patterned multi-creviced state. Such fractal-edged, multi-creviced surfaces as obtained by impaction were ultimately found herein to highly support and improve SERS applications in biomolecule detecting, even at single molecule level, and also in particular of communicable disease agent detection. (If for applications not relating to SERS, creation and shock-freezing these fractal-edged, multi-creviced agglomerates of nanoparticles would not be desired--which is not an objective of this application--it would simply suffice to heat the impaction surface to temperatures high enough (i.e. sufficiently close to the melting point of the metal in question, which in general far exceeds room temperature) to continue liquefaction and coalescence after impaction, as further depicted in figure 4). The P1 has an adjustable flow rate from 1-30 l/min for the carrier gas. The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors. For the experiments, the P1 settings were as follows: spark power of 1.3 kV and 10 mA; carrier gas was Argon at a speed of 1 liter/min for deposition; electrodes that were implemented were silver.

1 liter/min Sintering (or frittage) is the process of compacting and forming a solid mass of material by heat or pressure without melting it to the point of liquefaction, and may explain why the spherical particles as derived at by spark ablation surprisingly end up creating surfaces with increased detection sensitivity in SERS, as provided herein in this application. Sintering happens naturally in mineral deposits or as a manufacturing process used with metals, ceramics, plastics, and other materials. The atoms in the materials diffuse across the boundaries of the particles, fusing the particles together and connecting the material. The temperature does not have to reach the melting point of the material to achieve sintering. It is herein recognized that sintering nanoparticle metal likely contributes to a more frequent occurrence of so called "plasmon hot spots" in the surface of the substrate, and said increased frequency is thought a main origin of enhancement in surfaced-enhanced Raman scattering and detection, as found by the inventors. The method of deposition or printing nanoparticles seems to create a surface structure like a sponge and has been recognized herein to present improved hot spot surface morphology for applying SERS. Sintering of spherical particles in agglomerates is thought to follow fractal dimensions, possibly explaining why random deposition by impaction of ablated particles contributes to hot spot formation. Multiparticle sintering (Eggersdorfer et al., Langmuir 2011, 27, 10, 6358-6367) is encountered in almost all high temperature processes for material synthesis and energy generation (e.g., fly ash formation) resulting in fractal-like aggregates of primary particles (hard- or sinter-bonded agglomerates). Prolonged sintering or increased temperatures during sintering may compact the material and finally lead to a decrease in the fractal dimension of the agglomerates and loss of hot spot morphology, however, in general the present impaction process does not reach that stage.

Here we produce highly concentrated clouds of metal (pure or alloy), or metal compound vapors which are subsequently quenched at room temperature to form nanoparticles. A convenient way of doing this is by spark ablation, where microsecond discharges between electrodes produce particles of the electrode material in the atomic cluster or nanometer size range. These particles are then transported in a gas and focused deposited or broadly spread onto surfaces by impaction. In the impaction process, local temperature and pressure are raised sufficiently high to generate a sintering process that proceeds via fractal clustering to connect individual nanoparticles or already formed particle clusters through atomic diffusion and liquefaction. The impaction process is halted at the fractal cluster stage, leaving fractal-patterned and multi-creviced clusters of nanoparticles on the surface. If a solid surface is required rather than a porous material, for example for conductive purposes (and not for SERS, this can be achieved by increasing the temperature of the surface causing further sintering.

### EXPERIMENTAL SECTION

### CONTENT

1 MHV A59 and Coxsackievirus
2 Influenza A
3 H-CoV-NL63
4 Zika virus
5 Buffers & Mediums
6 SARS-CoV-2

### Example 1 MHV A59 and Coxsackievirus

### A. METHODS

*A.1 Sample preparation.* Samples containing Coxsackievirus B3 (10^7 PFU/ml) and Mouse hepatitis virus (MHV) A59 (10^8 PFU/ml) were obtained from the University of Utrecht. The samples were kept at -80 °C.

Substrates were produced by the company VSPARTICLE using their printer (VSP-P1). Silver lines of 1 mm x 3 mm were printed as SERS active area on ITO (indium tin oxide) coated glass slides (20 mm x 15 mm). The substrates (herein also indicated as chips) were kept in boxes sealed in vacuum bags until use.

Before measurement the Coxsackievirus B3 and MHV A59 samples were defrosted and diluted 1000x in methanol or ethanol (1) for inactivation. A quantity of 10 µl of the inactivated sample mixture was transferred to the SERS active surface on the substrate using a 10 µl pipette. The substrate was left to dry (2). After drying the Raman measurements started.

For measurements with a handheld spectroscope in the ML2 lab a spiked sample was made by mixing a negative patient sample (see chapter 2) with the MHV A59 (1:1). The MHV A59 virus and the negative patient were also measured. All samples were diluted 100x in ethanol.

*A.2 Instrumentation.* The P1 has an adjustable flow rate from 1-30 l/min for the carrier gas.

The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors.

For the experiments the P1 settings were as follows: spark power of 1.3 kV and 10 mA; carrier gas was Argon at a speed of 1 liter/min for deposition; electrodes that were implemented were silver. The samples were measured using a 514 nm wavelength laser 0.25 mW laser power and 10s acquisition time. The detection wavelength range was set on 200-2200 cm⁻¹. As a control, samples were measured on plain ITO coated glass. The Raman spectrum of MHV-A59 samples was also measured using the 785 nm laser on the same Renishaw spectroscope. The laser power was 0.106 mW during 10s acquisition time. The detection wavelength range was set on 200-2000 cm⁻¹. For handheld SERS spectra acquisition, the Mira DS Advanced (art. No. 2.926.0020, Metrohm) with a 785 nm laser with SERS A attachment and a spectral range of 400-2300 cm⁻¹ was used. Laser power was set to 1 using the Mira DS software (version 1.1.14) and acquisition time to 10s. For each sample both the Raster (3x) and no Raster (3x) setting were used.

### B. RESULTS

In figure 8: SERS spectrum of Coxsackievirus is shown

In figure 9: SERS spectra of MHV A59, human 4, and MHC A59 + Human 4 is shown

### C. DISCUSSION

**TABLE 1**

| Coxsackievirus | |
|---|---|
| Raman shift (cm⁻¹) | Band assignment |
| 650 | RNA |
| 764 | Tryptophan |
| 832 | Tyrosine |
| 915 | RNA |
| 974 | RNA |
| 1007 | Phenylalanine |
| 1156 | Protein |
| 1285 | RNA |

In table 1 and 2 the Raman shifts of the peaks of the Raman spectrum, shown in figure 8 and 9, are given. These peaks were assigned to their corresponding chemical bands. The spectrum of Coxsackievirus has distinct characteristic peaks as shown in table 1. The peaks 650 cm⁻¹ (Guanine), 915 cm⁻¹ (ribose vibration of RNA), 974 cm⁻¹ (ribose vibration of RNA) and 1285 cm⁻¹ (Cytosine) are related to RNA (3).The peak at 764 cm⁻¹ indicates presence of the amino acid tryptophan (3).The peak 832 cm⁻¹ is assumed from literature to be related to tyrosine (3).The peak 1007 cm⁻¹ is due to the in-plane C-H bending mode of phenylalanine(3,4). The peak at 1156 cm⁻¹ indicates is assumed to be related to the C-C and C-N stretching of proteins (3).

**TABLE 2**

| MHV A59 | |
|---|---|
| Raman shift (cm⁻¹) | Band assignment |
| 850 | Tyrosine |
| 1007 | Phenylalanine |
| 1030 | Phenylalanine |
| 1134 | C-C skeletal |
| 1244 | RNA |
| 1400 | Stretching of COO- |
| 1586 | Phenylalanine |

MHV A59 has characteristic peaks as shown in table 2. The peak at 850 cm⁻¹ is due to the C-C stretch in tyrosine (3,4). The peaks at 1007 cm⁻¹ (the in-plane C-H bending mode of phenylalanine (3,4)) and 1030 cm⁻¹ (symmetric ring breathing mode of phenylalanine (3,4))and 1586 cm⁻¹ (C=C bending mode of phenylalanine (3)) are related to the amino acid phenylalanine. The peak at 1134 cm⁻¹ (C-C skeletal change formation) is coming from the fatty acids of the lipid envelope. (3,4) The peak at 1244 cm⁻¹ is one of the most distinct peaks of RNA indicating the binding of RNA to the nanoparticles. The peak at 1400 cm⁻¹ is from the COO- stretching of protein amino acids close to the nanoparticles surface (3). Figure 9 shows the Raman spectra of MHV A59 and a human negative sample. It can be seen that the Raman spectra of the spiked human have the same peaks as the MHV A59 Raman spectra, therefore it can be said that a human negative sample does not hold back viral signals. The Limit of Detection (LoD) of the Coxsackievirus in figure 1 is 10^(4) PFU/ml, which corresponds to a 1000x dilution in methanol. The LoD of MHV A59 in figure 2 is 10^(5) PFU/ml, which corresponds to a 100x dilution in ethanol.

### Example 2 INFLUENZA (H1N1, H1 Hawaii, H1NL, H3N2)

### A. METHODS

*A.1 Sample preparation.* The following influenza A samples were acquired from Amsterdam Medical Centre (AMC): Hawaii 31 2007 H1WT TCID50/50ul = 4 (H1N1),; A/Hawaii/21/2007 H1 H274Y TCID50/50ul = 4 (H1); A/Ned/165108 2008-179 H1 (H1Ned); A/wisconsin/67/2005[S31N] H3N2 TCID50/50ul = 3 (H3N2); 3 throat swab samples each from patients found infected with infected with one of the influenza viruses. Samples were diluted in UTM viral transport medium (Copan); throat swab samples from patients not infected with influenza viruses in UTM were used to provide negative testing material

H1N1, H1, H1Ned and H3N2 viruses were propagated in MDCK (Madin-Darby Canine Kidney) cells according to AMC protocol and put in UTM for transporting to the laboratory. The samples were kept at -80 °C when not used (4).

AMC protocol: add 100 µl stock virus and 20 ml growth medium (EMEM 1x Minimum Essential Medium (Lonza LO BE-136f) 500 ml, FKS (Foetal calf serum) (Lonzo Cat.No. DE14-801F) 40 ml, NEAA 100x Non-Essential Amino Acids (Sciencell Cat.No. 0823) 5 ml, L-Glutamine 200mM in 0.85% NaCl (Lonza Cat.No.17-605E) 0.5 ml, Penicillin - Streptomycin each 10.000 U/ML (Lonzo DE17-602E) 5ml) to a growth bottle 75 cm^2 with a cell monolayer. Grow until cytopathological effect of 75-100%. Freeze on -80 °C. After 1d defrost, suspend and transfer 500 µl to eppendorf tubes (1.5ml). Determine the TCID50 for one of the tubes. Freeze the tubes at -80 °C again.

Substrates (chips) were produced using VSPARTICLE printer (P1). Silver lines of 1 mm x 3 mm were printed as SERS active area on ITO (indium tin oxide) coated glass slides (20 mm x 15 mm). The substrates were kept in boxes sealed in vacuum bags until use (5).

Before measurement, the samples were defrosted and diluted 100x in ethanol (EtOH 70%). A quantity of 10 µl of the sample mixture was transferred to the Ag nanolayer surface on the substrate using a 10 µl pipette. The Raman measurements started when the sample on the chip was dry.

*A.2 Instrumentation.* The P1 has an adjustable flow rate from 1-30 l/min for the carrier gas. The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors.

For the experiments, the P1 settings were as follows: spark power of 1.3 kV and 10 mA; carrier gas was Argon at a speed of 1 liter/min for deposition; electrodes that were implemented were silver. The SERS spectra were acquired on a Renishaw Raman system with a 785 nm laser and a maximum laser power of 10.6 mW at the Faculty of Aerospace Engineering of the TU Delft. For the SERS measurements a laser power of 0.106 mW was used with a 10s acquisition time (6). The lens magnification of 50x was used during the measurements.

*A.2 Data analysis.* The SERS spectra were baselined and normalized with software using Renishaw Wire 4.1. PCA was used for further analysis of the data, which is a method of recasting the high dimensional data onto a new set of axes or orthogonal basis vectors that are typically called principal components (PC) (7). More specifically, PCA will reduce the number of variables to the most dominant ones to group the data. Using the principal component analysis method, the purpose is to interpret the complex Raman spectra of different viruses by revealing differences between the samples (expressed as so-called "scores") and relating them to differences in the variables (called "loadings") defining a sample.

The Principle Component Analysis (PCA) model was constructed using the Principal Component Analysis for Spectroscopy tool on Origin Software (8). Before the analysis, the data were baseline corrected on Renishaw Wire software and normalized to highest intensity using Excel.

### RESULTS

In figure 10: SERS spectra of influenza A strains (H1 Hawaii, H1N1, H1NL and H3N2) are shown

In figure 11: Classification of H1 Haw, H1 NL, H1N1 and H3N2 SERS spectra by PCA is shown

In figure 12: Classification of positive and negative Influenza SERS spectra by PCA is shown.

### C. DISCUSSION

Influenza virus strains were shown to present differences in the SERS spectra specifically to their type. To further investigate the feasibility of using SERS using a chip according to the invention to differentiate different virus strains, PCA was conducted based on SERS spectra acquired from 4 Influenza virus samples (H1 Haw, H1 NL, H1N1 and H3N2) on range between 400-1360 cm⁻¹(Figure 4). For the PCA model a total of 44 spectra (by 11 replicants per each virus strain) were used and a two-dimensional score plot was created using the principle component 1 vs principal component 2. The score plots demonstrate the clustering of four different virus strains. For example, a large positive value for PC1 suggests the sample is likely to be H1 Haw while a negative score for PC 1 suggests the sample contains the H1 NL strain of Influenza. Similarly, a positive value for PC 2 indicates that the sample is likely to be H1N1 or H3N2. The combination of the information contained in PC1 and PC2 axes reveals a clear discrimination between virus strains, with the two first principal components explaining a 72.6% of the variations of the total data set. These results indicate that SERS technology with a chip according to the invention can be used to differentiate viruses at the strain level.

Furthermore, to make an effective classification of Influenza positive and negative samples, PCA was also used to establish statistically significant differences between these SERS spectra. The PCA model generated was used to classify the 110 spectra (4 influenza positive samples and 2 Influenza negative samples by 11 replicates each) in a range between 400-1360 cm⁻¹. Figure 5 illustrates a two-dimensional score plot that was created using the principle component 1 vs principal component 2. The score plots demonstrate a clear clustering between influenza human positive and negative specimens. More specifically, a positive value for PC1 suggests the sample is likely to be negative while a negative score for PC 1 suggests the samples are Influenza positive. The combination of the information contained in PC1 and PC2 axes reveals a clear discrimination between positive and negative samples, with the two first principal components explaining a 72.7% of the variations of the total data set. Classification results are direct evidence that our system can be employed as a rapid, direct virus detector.

**TABLE 3**

| Influenza A | | |
|---|---|---|
| Raman shift (cm⁻¹) | Band assignment | Type of influenza A (H1 Hawaii, H1N1, H1 NL and H3N2) |
| 535 | Protein | H1 NL, H3N2 |
| 663 | Cysteine | H1N1, H1 NL and H3N2 |
| 715 | Adenine | H1 Hawaii, H1N1, H1 NL and H3N2 |
| 764 | Tryptophan | H1 Hawaii, H1N1, H1 NL and H3N2 |
| 850 | Tyrosine | H1 Hawaii, H1N1, H1 NL and H3N2 |
| 867 | RNA | H1 Hawaii, H1N1, H1 NL and H3N2 |
| 915 | RNA | H3N2 |
| 974 | RNA | H1 Hawaii, H1N1, H1 NL and H3N2 |
| 1007 | Phenylalanine | H1 Hawaii, H1N1, H1 NL and H3N2 |
| 1030 | Phenylalanine | H1 Hawaii, H1N1, H1 NL and H3N2 |
| 1053 | C-N stretching | H1N1, H1 NL and H3N2 |
| 1134 | C-C skeletal | H1 NL and H3N2 |
| 1244 | RNA (T, A) | H1 Hawaii, H1N1, H1 NL and H3N2 |

In table 3 the Raman shifts of the peaks of the Raman spectrum, showed in figure 10, are given. These peaks are assigned to their corresponding chemical bands. Additionally, in the third row of table 3 it is illustrated which strains of the influenza A virus contain the Raman shift with chemical binding in their corresponding Raman spectrum. H1 Hawaii, H1N1, H1 NL and H3N2 show all peaks at 715 cm⁻¹ (Adenine C-N stretch (3)), 764 cm⁻¹ (tryptophan ring breathing (3)), 850 cm⁻¹ (tyrosine C-C stretch (3,4)), 867 cm⁻¹ (Ribose vibration of RNA(3)), 974 cm⁻¹ (Ribose vibration of RNA (3)), 1007 cm^{- 1} (the in-plane C-H bending mode of phenylalanine(3,4)), 1030 cm⁻¹ (symmetric ring breathing mode of phenylalanine(3,4)) and 1244 cm⁻¹ (One of the most distinct peaks of RNA (3)). It can be observed that peaks at the same Raman shift show different intensities for example the peak at 850 cm⁻¹ and 1007 cm⁻¹ has for the H1 Hawaii a lower intensity peak compared to the other Influenza A types. Besides the difference intensity there are also peaks visible for different types of Influenza A. The peaks at 535 cm⁻¹ (disulphide stretching mode of proteins) and 1134 cm⁻¹ (C-C skeletal change transformation) for H1 NL and H3N2 can distinguish them from H1 Hawaii and H1N1. (3,4). The peaks in H1N1, H1 NL and H3N2 at 663 cm⁻¹ (the C-S mode of the amino acid cysteine (3)) and 1050 cm⁻¹ (C-N stretch of proteins (3,4)) can distinguish them from H1 Hawaii. The peak at 915 cm⁻¹ (Ribose vibration of RNA) seems to be specific for H3N2. To conclude, the different types of influenza A show differences in the SERS spectra which make it possible to distinguish the different types. The Limit of Detection (LoD) of the influenza A virus strains are as follows: H1N1 and H1 Hawaii have a LoD of 0.4 TCID50/ml; H3N2 has a LoD of 0.03 TCID50/ml. The LoD values of the influenza A virus strains correspond to diluted samples of 100x in ethanol.

### Example 3 H-CoV-NL63

### A. METHODS

*A.1 Sample preparation.* From Amsterdam Medical Centre (AMC) the following samples were acquired: HCoV-NL63 HWE005 183 (216355182) 17-8-2016 TCID50 = 2.2 ul (NL63), UTM viral transport medium (Copan), Eswab medium (Copan), Lysis buffer (L6). Previously acquired patient samples from patients not infected with influenza A (described above) and MHV-A59 virus (University of Utrecht, described above) were used in the experiments with H-CoV-NL63. Spiked samples were made by mixing H-CoV-NL63 or MHV-A59 with the non-infected patient samples (1:1 and 1:10).

The H-CoV-NL63 virus was propagated in Rhesus Monkey Kidney Epithelial (LLC-MK2) cells according to AMC protocol described above (10). For transportation to our lab the viruses were put in UTM (Copan). They were kept at -80 °C when not used.

Substrates were produced by the company VSPARTICLE using their printer (P1). Five silver lines of 1 mm x 3 mm were printed such that a 3 mm x 3 mm SERS active area was created on ITO (indium tin oxide) coated glass slides (20 mm x 15 mm). The substrates were kept in boxes sealed in vacuum bags until use.

Before measurement the samples were defrosted and diluted 100x in ethanol (EtOH 70%). Samples were transferred to the substrates (10 µl) using a 10 µl pipette and were left to dry before measurement.

*A.2 Instrumentation.* The P1 has an adjustable flow rate from 1-30 l/min for the carrier gas.

The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors.

For the experiments the P1 settings were as follows: spark power of 1.3 kV and 10 mA; carrier gas was Argon at a speed of 1 liter/min for deposition; electrodes that were implemented were silver. The SERS spectra were acquired using a Mira DS Advanced (art. No. 2.926.0020, Metrohm) with a 785 nm laser with SERS A attachment and a spectral range of 400-2300 cm⁻¹. Laser power was set to 1 using the Mira DS software (version 1.1.14) and acquisition time to 10s. For each sample both the Raster (3x) and no Raster (3x) setting were used. After each measurement the substrate was replaced such that a new area was scanned.

*A.2 Data analysis.* The SERS spectra of H-CoV-NL63 were normalized using the Raman software of the Mira DS Advanced (art. No. 2.926.0020, Metrohm).

### B. RESULTS

In figure 13: SERS spectrum of H-CoV-NL63 is shown

In figure 14: SERS spectra of H-CoV-NL63 + human 4 is shown

### C. DISCUSSION

**TABLE 4**

| H-CoV-NL63 | |
|---|---|
| Raman shift (cm⁻¹) | Band assignment |
| 1007 | Phenylalanine |
| 1030 | Phenylalanine |
| 1053 | C-N/C stretching |
| 1134 | C-C skeletal |
| 1244 | RNA |
| 1359 | Tryptophan |
| 1400 | Stretching of COO- |
| 1586 | Phenylalanine |

In table 4 the Raman shifts of the peaks of the Raman spectrum, shown in figure 13, are given. These peaks are assigned to their corresponding chemical bands. H-CoV-NL63 shows characteristic peaks which can be related to specific components of the virus. The peaks at 1007 cm⁻¹ (the in-plane C-H bending mode of phenylalanine (3,4)) and 1030 cm⁻¹ (symmetric ring breathing mode of phenylalanine (3,4) and 1586 cm⁻¹ (C=C bending mode of phenylalanine (3)) are related to the amino acid phenylalanine. The peak at 1053 cm⁻¹ (C-N or C-O stretching) is assumed to be from the proteins (3,4). The peak at 1134 cm⁻¹ (C-C skeletal change formation) is coming from the fatty acids of the lipid envelope (3,4). The peak at 1244 cm⁻¹ is one of the most distinct peaks of RNA (3,4). The peak at 1359 cm⁻¹ is assumed to be from the amino acid tryptophan (3). The peak at 1400 cm⁻¹ is from the COO- stretching of protein amino acids close to the nanoparticles surface (3).

The Limit of Detection (LoD) of H-CoV-NL63 when diluted 100x in ethanol is 0.022 TCID50/ml

### Example 4 Zika virus

### A. METHODS

*A.1 Sample preparation.* Zika virus (Zika) stock solution in Dulbecco's Modified Eagle Medium with penicillin/streptomycin, glutamate (DMEM) (9 x 10^6 particles/ml) prepared by Artemis One Health was used. The Zika solution and DMEM were centrifuged on 10000 RPM for 5min (Labnet Microcentrifuge Technico Maxi, Fisher Scientific). The supernatant was transferred to an Eppendorf tube (1.5ml). The Zika supernatant was diluted 1x in ethanol, demi water and centrifuged medium. The Zika supernatant and all solvents were also measured

Substrates were produced by the company VSPARTICLE using their printer (P1). Five silver lines of 1 mm x 3 mm were printed such that a 3 mm x 3 mm SERS active area was created on ITO (indium tin oxide) coated glass slides (20 mm x 15 mm). The substrates were kept in boxes sealed in vacuum bags until use.

For SERS spectra acquisition, 10 µl sample was transferred to a substrate using a 10 µl pipette. For each sample one substrate was measured while the sample solution was still wet and one substrate was measured while the sample solution had dried.

*A.2 Instrumentation..* The P1 has an adjustable flow rate from 1-30 l/min for the carrier gas. The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors. For the experiments the P1 settings were as follows: spark power of 1.3 kV and 10 mA; carrier gas was Argon at a speed of 1 liter/min for deposition; electrodes that were implemented were silver. The SERS spectra were acquired using a Mira DS Advanced (art. No. 2.926.0020, Metrohm) with a 785 nm laser with SERS A attachment and a spectral range of 400-2300 cm⁻¹. Laser power was set to 1 using the Mira DS software (version 1.1.14) and acquisition time to 10s. For each sample both the Raster (3x) and no Raster (3x) setting were used. After each measurement the substrate was replaced such that a new area was scanned.

*A.2 Data analysis.* The P1 settings were as follows: spark power of 1.3 kV and 10 mA; carrier gas was Argon at a speed of 1 liter/min for deposition; electrodes that were implemented were silver. The SERS spectrum of Zika virus was normalized using the Raman software of the Mira DS Advanced (art. No. 2.926.0020, Metrohm).

### B. RESULTS

In figure 15: SERS spectrum of Zika virus is shown

### C. DISCUSSION

**TABLE 5**

| Zika virus | |
|---|---|
| Raman shift (cm⁻¹) | Band assignment |
| 850 | Tyrosine |
| 1007 | Phenylalanine |
| 1134 | C-C skeletal |
| 1285 | RNA |
| 1400 | Stretching of COO- |
| 1515 | RNA |
| 1597 | COOH |

In table 5 the Raman shifts of the peaks of the Raman spectrum, showed in figure 15, are given. These peaks are assigned to their corresponding chemical bands.

The peak at 850 cm⁻¹ is due to the C-C stretching of tyrosine. The peak 1007 cm⁻¹ is due to the in-plane C-H bending mode of phenylalanine (3,4). The peak at 1134 cm⁻¹ (C-C skeletal change formation) is coming from the fatty acids of the lipid envelope (3,4). The peaks at 1285 cm⁻¹ and 1515 cm⁻¹ is assumed to be from cytosine which can be found in RNA. The peak at 1400 cm⁻¹ is from the COO- stretching of protein amino acids close to the nanoparticles surface (3). The peak at 1597 cm⁻¹ is the COOH group on the amino acid side chains. The Limit of Detection (LoD) of the Zika virus when diluted in a 1:1 ratio with demiwater is equal to 5^{∗}10^5 particles/ml.

### Example 5 Buffers & Mediums

### A. METHODS

*A.1 Sample preparation.* For evaluating the spectra that were obtained from virus samples, the SERS spectra of the media had to be measured. This enabled identification of peaks specific to the virus (10). GLY/VTM medium was acquired from RIVM, UTM from AMC and RIVM, Eswab medium from AMC and DMEM-1 from Artemis One Health, Lysis buffer (L6) from AMC. The media were stored at room temperature.

Substrates were produced by the company VSPARTICLE using their printer (P1). Five silver lines of 1 mm x 3 mm were printed such that a 3 mm x 3 mm SERS active area was created on ITO (indium tin oxide) coated glass slides (20 mm x 15 mm). The substrates were kept in boxes sealed in vacuum bags until use.

*A.2 Instrumentation..* The P1 has an adjustable flow rate from 1-30 l/min for the carrier gas.

The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors.

For the experiments, the P1 settings were as follows: spark power of 1.3 kV and 10 mA; carrier gas was Argon at a speed of 1 liter/min for deposition; electrodes that were implemented were silver. The SERS spectra were acquired using a Mira DS Advanced (art. No. 2.926.0020, Metrohm) with a 785 nm laser with SERS A attachment and a spectral range of 400-2300 cm⁻¹. Laser power was set to 1 using the Mira DS software (version 1.1.14) and acquisition time to 10s. Measurement settings were left the same as with the virus measurement.

*A.3 Data analysis.* The SERS spectra of the buffer and mediums were normalized using the Raman software of the Mira DS Advanced (art. No. 2.926.0020, Metrohm).

### B. RESULTS

In figure 16: SERS spectrum of UTM (10 x diluted in ethanol) is shown

In figure 17: SERS spectrum of DMEM-1 is shown

In figure 18: SERS spectrum of GLY/VTM (10 x ethanol) is shown

In figure 19: SERS spectrum of Eswab medium is shown

In figure 20: SERS spectrum of Lysis buffer (L6) is shown

### C. DISCUSSION.

As is clear from the spectra, shown in the figures 16-20, various media contain Raman active molecules. The spectra of these media can potentially hide Raman signals from Raman signals of the virus molecules. By identifying which peaks are from the media, peaks can be attributed that are specific for the virus. Some media show noisier spectra than others. A noisier medium spectrum makes the peak analysis harder.

### Example 6 SARS-CoV-2

### A. METHODS

*A.1 Sample preparation.* From the Netherlands National Institute for Public Health and the Environment (RIVM) the following samples were obtained: three throat swab samples from patients infected with SARS-CoV-19 (Ct >30, 20-25 and 15-18), one throat swab sample from patient infected with influenza A (Ct 20-25), four throat swab samples from patients not infected with influenza A or SARS-CoV-19, SARS-CoV-2 stock solution in Dubecco's Modified Eagle Medium with penicilin/streptomycin, amphotericin B, nystatin (DMEM-2). Polymerase chain reaction (PCR) is a technique that is used to amplify the RNA of the virus. The Ct value is correlated to the amount of PCR in the reaction of this process, and a low Ct value corresponds to a high amount of PCR product. For each sample, 100 µl was transferred to GLY/VTM and 100 µl to UTM for transportation except for the unknown and SARS-CoV-2 stock. All samples were put on 60 °C for 1h by RIVM for inactivation.

Substrates were produced by the company VSPARTICLE using their printer (P1). Five silver lines of 1 mm x 3 mm were printed such that a 3 mm x 3 mm SERS active area was created on ITO (indium tin oxide) coated glass slides (20 mm x 15 mm). The substrates were kept in boxes sealed in vacuum bags until use. Before measurement some samples were centrifuged on 10000 RPM for 5min (Labnet Microcentrifuge Technico Maxi, Fisher Scientific). The supernatant was transferred to an Eppendorf tube (1.5 ml) and diluted 10x in ethanol and demi water. Samples that were not centrifuged were also diluted 10x in ethanol and demi water. For SERS spectra acquisition, 10 µl sample was transferred to a substrate using a 10 µl pipette. Each sample was measured while still wet and dried up on separate substrates. The ethanol diluted samples were measured when dry and with Raster on. The demi water diluted samples were measured still wet and with no Raster.

*A.2 Instrumentation.* The P1 has an adjustable flow rate from 1-30 l/min for the carrier gas.

The gas carriers Argon or Nitrogen are available. The particle size, which is in the range of 1 atom to 20 nm, can be tuned by choosing a spark power by adjusting the voltage and current in the range of 0-1.3 kV and 0-10 mA. Electrode material that can be inserted with a variety of metals or semiconductors.

For the experiments, the P1 settings were as follows: spark power of 1.3 kV and 10 mA; carrier gas was Argon at a speed of 1 liter/min for deposition; electrodes that were implemented were silver. The SERS spectra were acquired using a Mira DS Advanced (art. No. 2.926.0020, Metrohm) with a 785 nm laser with SERS A attachment and a spectral range of 400-2300 cm⁻¹. Laser power was set to 1 using the Mira DS software (version 1.1.14) and acquisition time to 10s. For each sample both the Raster (3x) and no Raster (3x) setting were used.

*A.2 Data analysis.* The SERS spectra of SARS-CoV-2 were normalized using the Raman software of the Mira DS Advanced (art. No. 2.926.0020, Metrohm). The PCA models, shown in figure 15 and 16 were constructed using the Principal Component Analysis for Spectroscopy tool on Origin Software (7). The PCA model showed in figure 24 is based on a different protocol. From the raw Raman spectra, the Raman shift range of 750-1800 cm⁻¹ was taken. The data was smoothened using Savitzky-Golay smoothening, at a window size of 15 and poly order of 2. This was implemented in the rampy library (Rampy is a Python library that aims at helping to process spectroscopic data, such as Raman, Infrared or XAS spectra. It offers, for instance, functions to subtract baselines as well as to stack, resample or smooth spectra. It aims at facilitating the use of Python in processing spectroscopic data and helps normalizing spectra, for exmple for inclusion in a database; 11). The baselines of the Raman spectra were removed using double reweighted penalized least squares and implemented in the rampy library. All values below zero were removed, because of the interest in peaks only. The data is normalized per spectrum by dividing the spectrum by the maximum value of the spectrum. All the data is in the domain of [0,1]. Class priors were made equal using a combination of under and over sampling. Undersampling is done using the cluster centroid algorithm implemented in the imblearn package (12). Undersampling is executed until a class imbalance of COVID_SAMPLES/OTHER_SAMPLES = 0.6. Oversampling is done using the SMOTE algorithm, which is also implemented in the imblearn package until equal class priors arise. PCA is implemented to reduce dimensionality, which describe 65% and 13% of the variance. Gaussian Naive Bayes classifier is implemented in the scikit-learn package (13) to classify the SARS-CoV-2 samples.

The barcodes that are constructed from the Raman spectra are by finding the most important peaks that correspond to the characteristics of the sample, by taking the second derivative from a single spectrum. This shows the most extreme slope changes of the Raman spectra and indicates where the highest intensity peaks are. Thereafter, a threshold is chosen to cut off all information below and above a certain Raman shift value. A binary barcode is left after this step, which shows the most extreme peaks of the corresponding Raman spectra. Hereby, a database is generated with binary barcodes, which can be used for the data analysis of PCA modelling described above.

### B. RESULTS

In figure 21: SERS spectra of SARS-CoV-2, H-CoV-NL63 and MHV a59 are shown

In figure 22: Classification of SARS-CoV-2, MHV A59 and H-CoV-NL63 coronaviruses spectra by PCA is shown

In figure 23: Classification of positive and negative SARS-CoV-2 SERS spectra by PCA is shown

In figure 24: Classification of SARS-CoV-2 in relation to 670 human negative samples and medium/buffer SERS spectra by PCA is shown.

In figure 25: Constructed barcodes of Raman spectrum of SARS-CoV-2 are shown

In figure 26: Constructed barcodes of Raman spectra of buffer and media are shown

In figure 27: Constructed barcodes of Raman spectra of human negative samples are shown.

### C. DISCUSSION

Similarly, to further investigate the feasibility of using SERS to differentiate different viruses, PCA was conducted based on SERS spectra acquired from 3 different coronaviruses samples (SARS-CoV-2, MHV A59, H-CoV-NL63) on range between 800-1760 cm⁻¹(Figure 21). For the PCA model a total of 9 spectra (by 3 replicants per each virus sample) were used and a two-dimensional score plot was created using the principle component 1 vs principal component 2. The score plots demonstrate the clustering of three different coronaviruses. More specifically, a positive value for PC1 suggests the sample is likely to be MHV A59 while a negative score for PC 1 suggests the sample contains SARS-CoV-2. Similarly, a positive value for PC 2 indicates that the sample is likely to be H-CoV-NL63. The combination of the information contained in PC1 and PC2 axes reveals a clear discrimination between virus strains, with the two first principal components explaining a 91.7% of the variations of the total data set. These results, shown in figure 22, indicate that the invention as provided herein can be used to differentiate between viruses of the same family type such as coronaviruses.

Moreover, to make an effective classification of SARS-CoV-2 positive and negative samples, PCA was also used to establish statistically significant differences between these SERS spectra. The PCA model generated was used to classify the 21 spectra (3 SARS-CoV-2 positive samples and 4 negative samples by 3 replicates each) in a range between 800-1700 cm⁻¹. The samples were diluted in EtOH and then centrifuged (raster on). Figure 23 illustrates a two-dimensional score plot that was created using the principle component 1 vs principal component 2. The score plots demonstrate a clear clustering between SARS-CoV-2 human positive and negative specimens. More specifically, a positive value for PC1 suggests the sample is likely to be negative while a negative score for PC 1 suggests the samples are SARS-CoV-2 positive.. The combination of the information contained in PC1 and PC2 axes reveals a clear discrimination between positive and negatives samples, with the two first principal components explaining a 82.6% of the variations of the total data set. These results are direct evidence that the detection system according to the invention can be employed as SARS-CoV-2 detection tool and the discrimination thereof.

**TABLE 6**

| SARS-CoV-2 | |
|---|---|
| Raman shift (cm⁻¹) | Band assignment |
| 650 | RNA |
| 814 | RNA |
| 850 | Tyrosine |
| 867 | RNA |
| 915 | RNA |
| 974 | RNA |
| 1007 | Phenylalanine |
| 1030 | Phenylalanine |
| 1134 | C-C skeletal |
| 1216 | Stretching of C-N and |
| | Stretching of C-C₆H₅ |
| 1244 | RNA |
| 1285 | RNA |
| 1400 | Stretching of COO- |
| 1448 | CH deformation |
| 1461 | CH₂ deformation |
| 1506 | RNA |
| 1586 | Phenylalanine |

In table 6 the Raman shifts of the peaks of the Raman spectrum, showed in figure 21a, are given. These peaks are assigned to their corresponding chemical bands.

SARS-CoV-2 show characteristic peaks, the peaks 650 cm⁻¹ (Guanine (3)), 814 cm⁻¹ (one of the most distinct peaks of RNA), 867 cm⁻¹ (ribose vibration of RNA), 915 cm⁻¹ (ribose vibration of RNA), 974 cm⁻¹ (ribose vibration of RNA), 1244 cm⁻¹ (one of the most distinct peaks of RNA), 1285 cm⁻¹ (cytosine), and 1506 cm⁻¹ (N=H bending of cytosine) are peaks related to RNA. The peak at 850 cm⁻¹ is due to C-C stretch of the amino acid tyrosine. The peaks at 1007 cm⁻¹ (the in-plane C-H bending mode of phenylalanine (3,4)) and 1030 cm⁻¹ (symmetric ring breathing mode of phenylalanine (3,4)) and 1216 cm⁻¹ (C-N and C-C₆H₅ stretching) and 1586 cm⁻¹ (C=C bending mode of phenylalanine (3)) are related to the amino acid phenylalanine. The peak at 1400 cm⁻¹ is from the COO- stretching of protein amino acids close to the nanoparticles surface (3). The peak at 1448 cm⁻¹ is due to the CH deformation of phenyl groups (3). The peak at 1461 cm⁻¹ is the CH₂ deformation of lipids which indicates the lipid envelope (3).

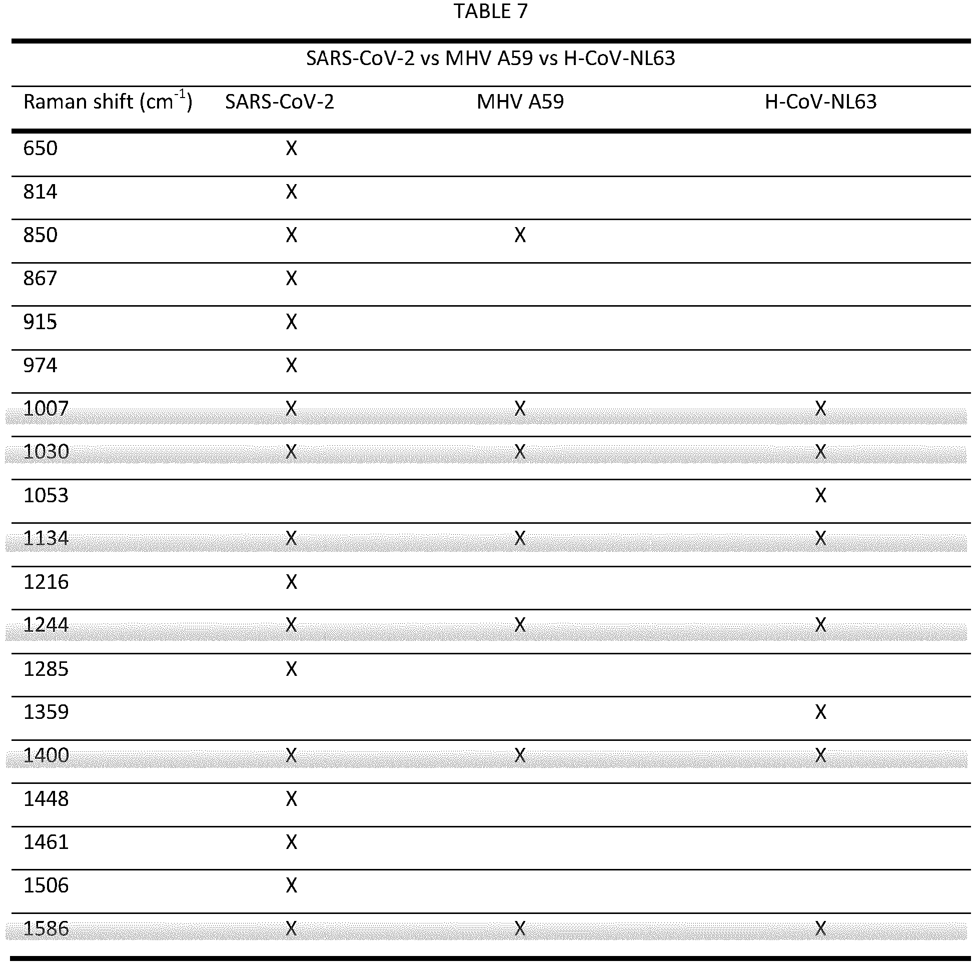

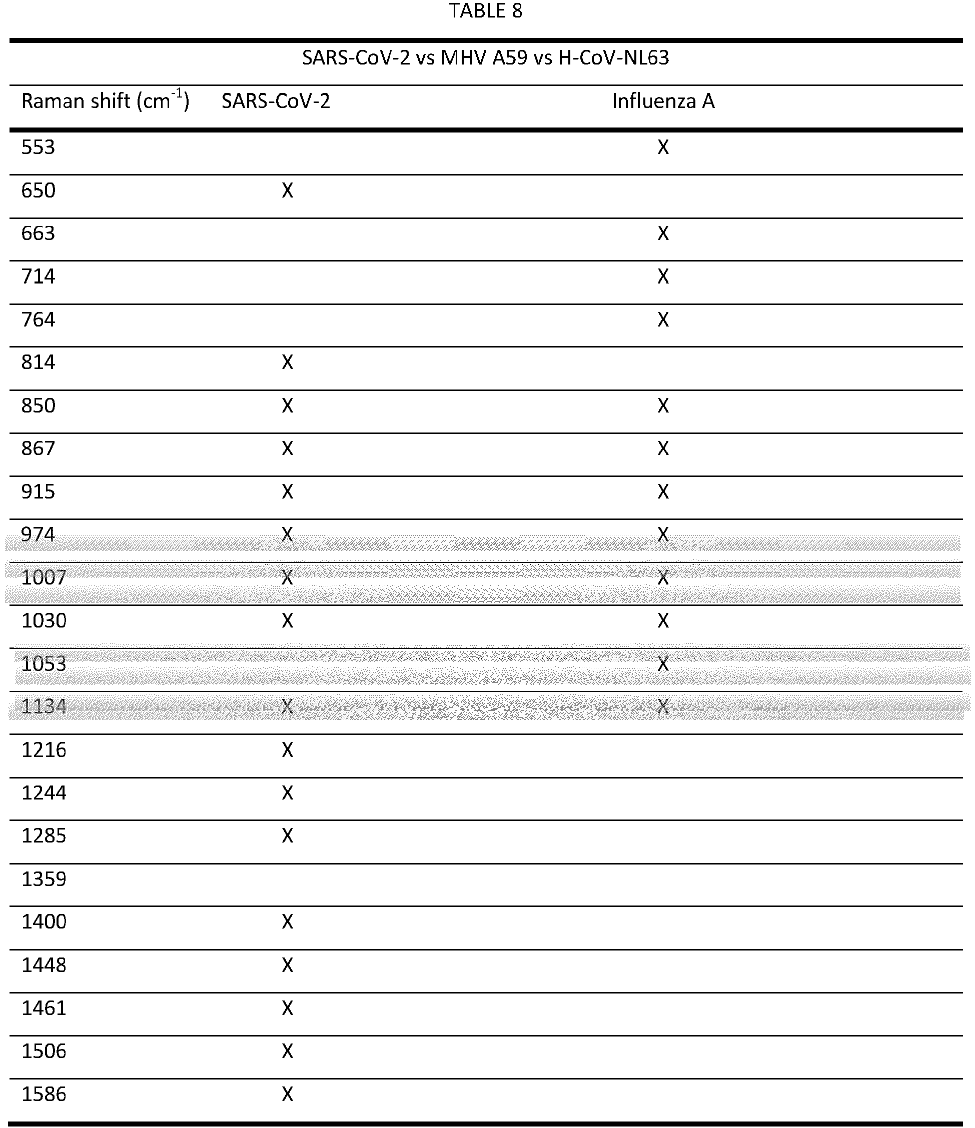

A comparison between Raman shifts of chemical bindings of the viruses SARS-CoV-2, MHV A59 and H-CoV-NL63, which are derived from their corresponding Raman spectra, is illustrated in table 7. The grey marked rows in the tables refer to a correlation, where they have the same Raman shift in their corresponding Raman spectra. It can be observed from table 7 that the viruses have their own characteristic peaks, which makes the Raman spectra unique for the viruses. The uniqueness allows the data analysis to keep the Raman spectra of different viruses distinguished. In table 8, a comparison between Raman shifts of chemical bindings of the viruses SARS-CoV-2 and influenza A is given. It can be observed that several peaks are similar in the Raman spectra of both viruses, and several peaks refer to their own characteristics. In conclusion, the Raman spectra that are acquired from the prepared virus samples can be distinguished.

Figure 24 shows a classification of SARS-CoV-2, human negatives and medium/buffer SERS spectra by a PCA model. Using 3-fold cross validation a result of 100% True negative and 100% True positive is consistently acquired. In figure 25, 26 and 27, the barcodes of SARS-CoV-2, buffer, mediums and negative human samples are illustrated. These barcodes are saved in the database and can be used for the data analysis to be able to quickly classify new samples. The binary barcodes analysis, provide more specific and quicker classification skills to the system. Suitable overall Raman shift peak assignments for addition to said database, allowing comparing and classification are shown in table 9

| Table 9, overview of Raman shift peak assignments for inclusion in a database of a Raman spectroscopic device according to the invention. | Figure reference | Table reference |
|---|---|---|
| Coxsackievirus | 8 | 1 |
| MHV A59, human 4 and MHC A59 + Human 4 | 9 | 2 |
| Influenza A strains (H1 Hawaii, H1N1, H1NL and H3N2) | 10 | 3 |
| H-CoV-NL63 | 13 | 4 |
| H-CoV-NL63 + human 4 | 14 | |
| Zika virus | 15 | 5 |
| UTM (10x ethanol) | 16 | |
| DMEM-1 | 17 | |
| GLY/VTM (10x ethanol) | 18 | |
| Eswab medium | 19 | |
| Lysis buffer (L6). | 20 | |
| SARS-CoV-2, H-CoV-NL63 and MHV a59. | 21 | 6, 7 and 8 |
| Constructed barcodes SARS-CoV-2 | 25 | |
| Constructed barcodes of Raman spectra of buffer and media | 26 | |
| Constructed barcodes of Raman spectra of human negative samples | 27 | |
| Human cellular organelles | 28 | |
| Biomolecules extracted from cells | 29 | |
| Microplastics | 30 | |
| MRSA | 31 | |
| Fentanyl | 32 | |
| Mycoplasma | 33 | |
| Malaria in red blood cells | 34 | |
| Fungal pathogens from banana | 35 | |
| Malaria | 36 | |
| Estrogen | 37 | |
| Glucose | 38 | |
| Pesticide | 39 | |
| Antibodies | 40 | |
| Fungal pathogens | 41 | |
| Bacterial pathogens | 42 | |
| Psychoactive substances | 43 | |
| Prions | 44 | |

Suitable elements for particle generation, and suitable elements for carrier gas are shown in Table 10.

**TABLE 10**

| Suitable elements for particle generation and elements for carrier gas | | | |
|---|---|---|---|
| Element particle generation | | | Element carrier gas |
| Lithium | Molybdenum | Aluminium | Nitrogen |
| Beryllium | Tungsten | Gallium | Oxygen |
| Sodium | Manganese | Indium | Hydrogen |
| Magnesium | Rhenium | Thallium | Helium |
| Potassium | Iron | Carbon | Neon |
| Calcium | Ruthenium | Silicon | Argon |
| Scandium | Osmium | Germanium | Krypton |
| Rubidium | Cobalt | Tin | Xenon |
| Strontium | Rhodium | Lead | |
| Cesium | Iridium | Arsenic | |
| Barium | Nickel | Antimony | |
| Yttrium | Palladium | Bismuth | |
| Titanium | Platinum | Tellurium | |
| Zirconium | Copper | All Lanthanoids | |
| Hafnium | Silver | | |
| Vanadium | Gold | | |
| Niobium | Zinc | | |
| Tantalum | Cadmium | | |
| Chromium | Boron | | |

Table 11 Example of SERS detection time required in testing samples from the field.

| Table 11 Sample detection time | Total measurement time (s) | Average measurement time (s) per sample |
|---|---|---|
| 96 Negative SARS-CoV-2 | 7200 sec | 75 sec |
| 96 Positive SARS-CoV-2 | 7200 sec | 75 sec |

### REFERENCES with experimental section

1. Kampf, G. et al. Persistence of coronaviruses on inanimate surfaces and their inactivation with biocidal agents
2. Fan, C., Hu, Z., Riley, L. K., Purdy, G. A., Mustapha, A., & Lin, M. (2010). Detecting Food- and Waterborne Viruses by Surface-Enhanced Raman Spectroscopy. Journal of Food Science
3. Raman Spectroscopy of Biological Tissues: Applied Spectroscopy Reviews: Vol 42, No 5
4. Gould, E.A., Methods for long-term virus preservation. Molecular Biotechnology, 1999. 13(1): p. 57-66
5. Michieli, N., et al., Oxidation effects on the SERS response of silver nanoprism arrays. RSC Advances, 2017. 7(1): p. 369-378
6. Lin, Y.-Y., et al., Target-size embracing dimension for sensitive detection of viruses with various sizes and influenza virus strains. Biosensors and Bioelectronics, 2012. 35(1): p. 447-451
7. See Esbensen, K. H. Multivariate Data Analysis-in practice, 5 ed.: CAMO Process: Oslo, 2004
8. Originlab.com. 2020. Principal Component Analysis For Spectroscopy - File Exchange - Originlab. [online] Available at: <https://www.originlab.com/FileExchange/details.aspx?fid=326>
9. Abdul-Rasool S, Fielding BC. Understanding Human Coronavirus HCoV-NL63. Open Virol J. 2010;4:76-84. Published 2010 May 25. doi:10.2174/1874357901004010076
10. Premasiri WR, Gebregziabher Y, Ziegler LD. On the difference between surface-enhanced raman scattering (SERS) spectra of cell growth media and whole bacterial cells. Appl Spectrosc. 2011;65(5):493--499. doi:10.1366/10-06173
11. https://pypi.org/project/rampy/
12. https://pypi.org/project/imbalanced-learn/
13. https://scikit-learn.org/

## Claims

1. A chip conditioned for surface-enhanced Raman spectroscopic detection of at least one biomolecule present in a sample, said chip having a support of which a solid surface at least partly is provided with a fractal-patterned multi-creviced sintered agglomerate of metal nanoparticles and/or metal compound nanoparticles.

2. A chip for surface-enhanced Raman spectroscopic detection of at least one biomolecule present in a sample, said chip having a support of which a solid surface at least partly is provided with a fractal-patterned multi-creviced sintered agglomerate of metal nanoparticles and/or metal compound nanoparticles obtainable through spark ablation and/or impaction deposition.

3. A chip according to claim 1 or 2 wherein an average distance between nanoparticles or compositions thereof (or metal compound nanoparticles) is at most 32 nm, more preferably at most 16 nm, more preferably at most 8 nm, more preferably at most 4 nm, more preferably at most 2 nm, from each other.

4. A chip according to anyone of claims 1-3 wherein the size of said particles ranges from about 1nm to at about 20 nm, more preferably ranges from about 1nm to at about 10 nm, more preferably ranges from about 1nm to at about 7 nm, size most preferably ranges from about 3 to about 5nm. A particularly useful particle size is at around 4nm.

5. A chip according to anyone of claims 1-4 wherein said metal is selected from the group of silver, nickel, aluminium, silicon, gold, platinum, palladium, titanium, copper, cobalt, zinc and combinations thereof, and/or selected from an alloy of at least two metals selected from said group, or wherein said metal compound comprises an oxide, nitride, silicide, phosphide, oxynitride, or carbide of said metal and combinations thereof.

6. A chip according to anyone of claims 1-5, where in the support comprises a material selected from the group of silica, alumina, polymer and paper, and combinations thereof.

7. A chip according to anyone of claims 1-6, wherein said support comprises an Indium Tin Oxide (ITO) top layer upon which said agglomerate is at least partly provided.

8. A chip according to anyone of claims 1-7 wherein said agglomerate is deposited in a spot surface size of at least 1 square millimeter, preferably at least 4 square millimeters, more preferably at least 9 square millimeters.

9. A chip according to anyone of claims 1-8, wherein said agglomerate is deposited with a layer thickness of between 100 - 1500 nm, more preferably between 200 and 1100 nm, more preferably between 300 and 800 nm, most preferably between 450 and 650 nm

10. A chip according to anyone of claims 1-9, wherein the support is provided with a surface dimension of at least 5x5, preferably at least 10x10 millimeter.

11. A chip according to anyone of claims 1-10 wherein said biomolecule originates from a micro-organism, bacterium or communicable disease agent, such as a virus, such as a coronavirus.

12. A detection system for detecting by surface-enhanced Raman spectroscopy of at least one biomolecule present in a sample, comprising
a. a chip according to anyone of claims 1-11 for carrying the sample,
b. means for measuring a Raman scattered light signal or spectrum of the sample present on said detection chip,
c. means for collecting and processing the measured light signal or spectrum, and comparing the analysed data with data characteristic for the biomolecule,
d. means for determining based on the compared data the presence of absence of the biomolecule in the sample.

13. A kit of parts for detecting at least one biomolecule present in a sample, comprising
a. a chip according to anyone of claims 1-11 for carrying the sample,
b. a Raman spectroscopic device for measuring a Raman scattered light signal or spectrum of the sample on said detection chip,
c. a device for collecting and processing the light signal or spectrum and comparing the analysed data with data characteristic for the biomolecule, and
d. a device for determining based on the compared data the presence of absence of the biomolecule in the sample.

14. A method for testing a sample for the presence of a biomolecule in a detection system according to claim 13, comprising obtaining a biological sample, preferably a human sample, more preferably a nose or throat swab sample, dissolving this sample in a medium, placing an aliquot of the diluted sample on said chip on top of said fractal-patterned multi-creviced sintered agglomerate of metal nanoparticles and/or metal compound nanoparticles, subsequently placing the chip in said means for measuring a Raman scattered light signal or spectrum of said system, subjecting said aliquot to light, determining the light signal or spectrum of said aliquot and comparing the analysed data with a data characteristic for the biomolecule, allowing determination in said sample the presence of the biomolecule .

15. A method according to claim 14 wherein the medium is a volatile medium, such as 70% ethanol, and drying the diluted sample after placing the aliquot on the chip, and preferably the said testing is achieved in less than 60 minutes, facilitating homeland security use.

16. A method according to claim 14, wherein the medium is an aqueous non-volatile, such as demi water
, wherein said testing is achieved in less than 45 minutes, facilitating medical screening use.

17. A Raman spectroscopic device for measuring a Raman scattered light signal or spectrum provided with a chip according to any of claims 1-11, preferably said device is also provided with a database provided with Raman shift peak assignments, said assignments preferably selected from table 9, more preferably selected from an assignment or assignments provided for a virus, most preferably selected for a coronavirus.

18. A handheld device according to claim 17.

19. A method for making a chip conditioned for surface-enhanced Raman spectroscopic detection of at least one biomolecule present in a sample, said chip having a support of which a solid surface at least partly is provided with a fractal-patterned multi-creviced sintered agglomerate of metal nanoparticles and/or metal compound nanoparticles, said method comprising generating a flow of said nanoparticles in carrier gas in a vacuum , with a nanoparticle generator, provided with two metal (compound) electrodes with spark power sufficient to generate nanoparticles and depositing said nanoparticles on a solid surface wherein said deposition occurs by impacting said nanoparticles on said surface trough directing said flow in an angle perpendicular to said surface.

20. A method according to claim 19, said gas being argon, said flow set at a speed of 1 to 30 l/min, preferably 1 l/min, said spark power set at 0.1 to 1.3 kV, preferably 1.3 kV and 1 mA to 10 mA, preferably 10 mA; and said metal silver.

21. A method according to claim 19 or 20, said surface being an on ITO (indium tin oxide) coated glass slide, preferably with dimension 20x15 mm

22. A chip obtainable with a method according to anyone of claims 19 to 21.

23. Use of a chip according to claim 22 in a method according to anyone of claims 14-17.
